# EUROPEAN PATENT SPECIFICATION

(11) **EP 4 505 983 B1**
(45) Date of publication and mention of the grant of the patent: **06.05.2026**
(21) Application number: 23827005.2
(22) Date of filing: 08.06.2023
(51) Int. Cl.: A61F 13/49, A61F 13/496, A61F 13/51

(54) **UNDERPANTS-TYPE ABSORBENT ARTICLE**
SAUGFÄHIGER ARTIKEL VOM UNTERHOSEN-TYP
ARTICLE ABSORBANT DE TYPE SOUS-VÊTEMENT

(30) Priority: 20.06.2022 JP 2022099153
(43) Date of publication of application: 12.02.2025
(73) Proprietor: Unicharm Corporation, Ehime 799-0111 (JP)
(72) Inventor: OHTSUBO, Toshifumi, Kanonji-shi, Kagawa 769-1602 (JP); KAWAZU, Fumihito, Kanonji-shi, Kagawa 769-1602 (JP)
(74) Representative: Dolleymores
(86) International application number: PCT/JP2023/021407
(87) International publication number: WO 2023/248818

(56) References cited:
- EP-A1- 3 730 110
- WO-A1-2019/069807
- WO-A1-2019/123549
- WO-A1-2020/217745
- JP-A- 2019 063 411
- JP-A- 2019 170 831
- JP-A- 2019 170 831
- JP-A- 2020 179 047
- JP-B1- 7 066 900

## Description

### FIELD

The present invention relates to an underpants-shaped absorbent article.

### BACKGROUND

Conventionally, an underpants-shaped absorbent article has been widely known in which a front waist portion and a back waist portion are connected by side joining portions provided in two end portions in the lateral direction to form a waist opening and a pair of leg openings. For example, Patent Document 1 discloses an underpants-shaped disposable diaper having a front waist area (front panel), a back waist area (back panel) and a crotch area, and formed by joining the side edge portions of the front panel and the side edge portions of the back panel, for example, with semicircular joining spots.

### [CITATION LIST]

### [PATENT LITERATURE]

[Patent Document 1] Japanese Patent Application Publication No. 2015-96192
[Patent Document 2] EP3730110A1

### SUMMARY

### [TECHNICAL PROBLEM]

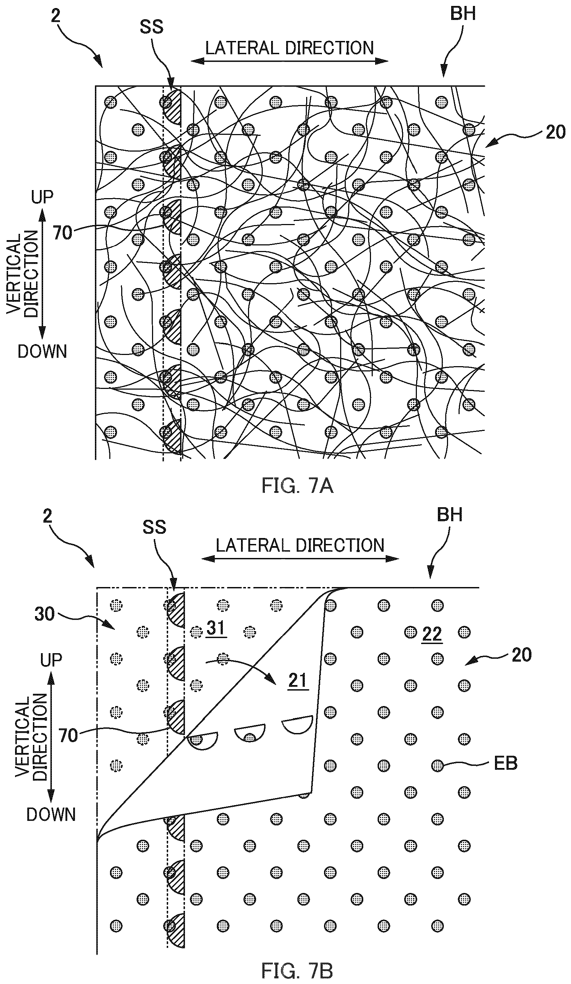

After using the underpants-shaped absorbent article, when removing it from the wearer's body (taking off the underpants-shaped absorbent article that is being worn), it is common to tear the waist area at the side joining portion to open the waist opening. When attempting to open the waist opening using the side joining portions of a disposable diaper such as ones described in Patent Document 1, the portion of the one-side waist area (e.g., front waist portion) that is joined by the side joining portion remains joined to the other-side waist area (e.g., back waist portion), and after tearing, one-side waist area may be left with the portion of the side joining portion cut out (see FIG. 7B). In other words, in the action of tearing the side joining portion to open the waist opening, the one-side waist area is torn along the contour of the side joining portion, which may require a force for strongly pulling the one-side waist area or may result in the one-side waist area tearing in an unintended part. On the other hand, if the side joining portion is provided on both side edges in the waist area continuously over the entire area in the vertical direction, it is possible to reduce the risk of the one-side waist area tearing in an unintended part. But, the both side edge portions of the waist area will become stiff, causing a risk that the wearer feel discomfort. Patent Document 2 discloses another pull-on garment which can be easily removed.

The present invention was achieved in light of conventional problems such as that described above, an aspect of the present invention is to provide an underpants-shaped absorbent article that is easy to tear along the joining portion after use, while reducing the risk of a joining region at a side end portion of the waist portion becoming excessively stiff.

### [SOLUTION TO PROBLEM]

A main aspect of the present invention of achieving the above-described aspect is an underpants-shaped absorbent article having a vertical direction, a lateral direction, and a front-back direction that intersect each other, the underpants-shaped absorbent article including: a pair of waist portions; and a liquid-absorbent absorbent body, the pair of waist portions being joined to each other by a pair of joining regions at both side end portions in the lateral direction, the joining regions each having a plurality of joining portions extending along the vertical direction, the waist portions including a one-side sheet and an other-side sheet, at least either one of the one-side sheet and the other-side sheet having a plurality of heat-fusing portions in which heat-fusible fibers are joined to each other, a heat-fusing-portion row extending along the vertical direction being formed by a group of the heat-fusing portions that are adjacent in the vertical direction in the stretched state and in which a distance of centers in the lateral direction is smallest in the stretched state, the heat-fusing portion not joining the one-side sheet and the other-side sheet, the heat-fusing-portion row having a first heat-fusing portion and a second heat-fusing portion that are located inside the joining portions in the lateral direction, a vertical gap between the joining portions being wider than a vertical length of the joining portion, the vertical gap between the joining portions being narrower than a gap between the first heat-fusing portion and the second heat-fusing portion.

Features of the present invention other than the above will become clear by reading the description of the present specification with reference to the accompanying drawings.

### [ADVANTAGEOUS EFFECT OF THE INVENTION]

According to the present invention, it is easier to tear along the lateral inner ends of the joining portions after use while reducing the risk of the joining region becoming excessively stiff, compared with the case where the gap in the vertical direction between the joining portions is wider than the length of the joining portion in the vertical direction and where the gap in the vertical direction between the joining portions is not narrower than the gap between the first heat-fusing portion and the second heat-fusing portion.

### BRIEF DESCRIPTION OF DRAWINGS

FIG. 1 is a schematic perspective view of a diaper **1.**
FIG. 2 is a schematic plan view of the diaper 1 in an unfolded and stretched state when viewed from the skin side of a wearer.
FIG. 3 is a cross-sectional view taken along line A-A in FIG. **2****.**
FIG. 4 is a diagram illustrating weld-portion rows R provided in a front waist portion 20.
FIGS. 5A and 5B are illustrative diagrams of attachment of waist elastic members 23.
FIG. 6 is a schematic enlarged view of a portion Y in FIG. 4.
FIG. 7A is a diagram illustrating the configuration of a conventional underpants-shaped absorbent article **2.**
FIG. 7B is a diagram illustrating a state of tearing the conventional underpants-shaped absorbent article **2.**
FIG. 8 is a diagram for explaining the schematic enlarged view of FIG. 6.
FIG. 9 is a diagram illustrating how the diaper 1 is torn.
FIG. 10A is a diagram illustrating weld-portion rows Ra and Rb of a modified example.
FIG. 10B is a diagram illustrating an interrupted-weld-portion pair 51a.
FIG. 11A is a diagram illustrating the interrupted-weld-portion pair 51a in the stretched state.
FIGS. 11B and 11C are diagrams illustrating the interrupted-weld-portion pair 51a in a contracted state.
FIG. 12 is a diagram showing a modified example of a heat-fusing portions EB.
FIG. 13 is a schematic plan view, with a partial perspective view, showing the process of manufacturing the diapers 1 in a manufacturing line 100.
FIG. 14 is a diagram illustrating a processing performed at a fourth processing position PK4.

### DESCRIPTION OF EMBODIMENTS

At least the following matters will be clear with the description of this specification and the attached drawings.

Aspect 1 is an underpants-shaped absorbent article having a vertical direction, a lateral direction, and a front-back direction that intersect each other, the underpants-shaped absorbent article including: a pair of waist portions; and a liquid-absorbent absorbent body, the pair of waist portions being joined to each other by a pair of joining regions at both side end portions in the lateral direction, the joining regions each having a plurality of joining portions extending along the vertical direction, the waist portions including a one-side sheet and an other-side sheet, at least either one of the one-side sheet and the other-side sheet having a plurality of heat-fusing portions in which heat-fusible fibers are joined to each other, a heat-fusing-portion row extending along the vertical direction being formed by a group of the heat-fusing portions that are adjacent in the vertical direction in the stretched state and in which a distance of centers in the lateral direction is smallest in the stretched state, the heat-fusing portion not joining the one-side sheet and the other-side sheet, the heat-fusing-portion row having a first heat-fusing portion and a second heat-fusing portion that are located inside the joining portions in the lateral direction, a vertical gap between the joining portions being wider than a vertical length of the joining portion, the vertical gap between the joining portions being narrower than a gap between the first heat-fusing portion and the second heat-fusing portion.

According to aspect 1, it is easier to tear along the lateral inner ends of the joining portions after use while reducing the risk of the joining region becoming excessively stiff, compared with the case where the gap in the vertical direction between the joining portions is wider than the length of the joining portion in the vertical direction and where the gap in the vertical direction between the joining portions is not narrower than the gap between the first heat-fusing portion and the second heat-fusing portion.

Aspect 2 is the underpants-shaped absorbent article according to the aspect 1, wherein a vertical length of the joining portion is shorter than a vertical length of the first heat-fusing portion.

According to aspect 2, it is possible to further reduce the risk of the joining region becoming excessively stiff compared with the case where the vertical length of the joining portion is longer than the vertical length of the first heat-fusing portion.

Aspect 3 is the underpants-shaped absorbent article according to the aspect 1 or 2, wherein a lateral length of the joining portion is longer than a vertical length of the joining portion.

According to aspect 3, it becomes easier to tear the absorbent article along the lateral inner ends of the joining portions after use while making it easier to increase the joining strength in the joining portions compared with the case where the vertical length of the joining portion is shorter than the lateral length of the joining portion.

Aspect 4 is the underpants-shaped absorbent article according to any one of the aspects 1 to 3, wherein a plurality of elastic members that can stretch and contract in the lateral direction are arranged at intervals in the vertical direction, between the one-side sheet and the other-side sheet, and a vertical length of the joining portion is shorter than a thickness of the elastic member in the natural state.

According to aspect 4, it is possible to reduce the possibility that the joining portion cuts the elastic member.

Aspect 5 is the underpants-shaped absorbent article according to any one of the aspects 1 to 4, wherein a plurality of elastic members that can stretch and contract in the lateral direction are arranged at intervals in the vertical direction, between the one-side sheet and the other-side sheet, and a vertical length of the joining portion is shorter than a thickness of the elastic member in the stretched state.

According to aspect 5, it is possible to further reduce the possibility that the joining portion cuts the elastic member.

Aspect 6 is the underpants-shaped absorbent article according to any one of the aspects 1 to 5, wherein in at least one of the waist portions, a plurality of elastic members that can stretch and contract in the lateral direction are arranged at intervals in the vertical direction, between the one-side sheet and the other-side sheet joined by a plurality of weld portions, the plurality of weld portions include a plurality of weld-portion pairs that are located on both sides of the elastic member in the vertical direction, when each of the elastic members contracts in the lateral direction, a position of the elastic member in the vertical direction relative to the one-side sheet and the other-side sheet is restricted by being sandwiched between the weld-portion pair, and a lateral length of the joining portion is longer than a lateral length of the weld portion.

According to aspect 6, it is easier to increase the strength of the joining region compared with the case where the lateral length of the joining portion is made shorter than the lateral length of the weld portion.

Aspect 7 is the underpants-shaped absorbent article according to any one of the aspects 1 to 6, wherein in at least one of the waist portions, a plurality of elastic members that can stretch and contract in the lateral direction are arranged at intervals in the vertical direction, between the one-side sheet and the other-side sheet joined by a plurality of weld portions, the plurality of weld portions include a plurality of weld-portion pairs that are located on both sides of the elastic member in the vertical direction, when each of the elastic members contracts in the lateral direction, a position of the elastic member in the vertical direction relative to the one-side sheet and the other-side sheet is restricted by being sandwiched between the weld-portion pair, the plurality of weld-portion pair includes a first weld-portion pair and a second weld-portion pair located adjacent to the first weld-portion pair on a one side in the lateral direction, the first weld-portion pair and the second weld-portion pair sandwich a first elastic member between them, the first weld-portion pair has a first weld portion and a second weld portion, the first weld portion and the second weld portion being located on the same side in the vertical direction with respect to the first elastic member and spaced with a first gap in the lateral direction, and in the stretched state, a first length is longer than a lateral length of the first gap, the first length being a lateral length from a lateral one-side end of the first weld-portion pair to a lateral other-side end of the second weld-portion pair.

According to aspect 7, it is possible to reduce the risk of the elastic member coming off from between the weld-portion pair, making it possible to suppress the decrease in stretchability due to the elastic member.

Aspect 8 is the underpants-shaped absorbent article according to any one of the aspects 1 to 7, wherein a gap between the first heat-fusing portion and the second heat-fusing portion is greater than twice a sum of a vertical length of the joining portion and the vertical gap between the joining portions.

According to aspect 8, it becomes easier to tear the absorbent article along the lateral inner end of the joining portion after use compared with the case where the gap between the first heat-fusing portion and the second heat-fusing portion is narrower than twice the sum of the vertical length of the joining portion and the vertical gap between the joining portions.

Aspect 9 is the underpants-shaped absorbent article according to any one of the aspects 1 to 8, wherein the vertical gap between the joining portions is narrower than a lateral length of the joining portion.

According to aspect 9, it becomes easier to tear the absorbent article along the lateral inner ends of the joining portions after use while making it easier to increase the joining strength in the joining portion compared with the case where the vertical gap between the joining portions is wider than the lateral length of the joining portion.

Aspect 10 is the underpants-shaped absorbent article according to any one of the aspects 1 to 9, wherein a lateral length of the joining portion is longer than a lateral length of the first heat-fusing portion.

According to aspect 10, it is possible to increase the joining strength of the joining region compared with the case where the lateral length of the joining portion is shorter than the lateral length of the first heat-fusing portion.

Aspect 11 is the underpants-shaped absorbent article according to any one of the aspects 1 to 10, wherein an area of the joining portion is equal to or less than an area of the first heat-fusing portion.

According to aspect 11, it is possible to reduce the risk of the joining region becoming excessively stiff compared with the case where the area of the joining portion is larger than the area of the first heat-fusing portion.

Aspect 12 is the underpants-shaped absorbent article according to any one of the aspects 1 to 10, wherein an area of the joining portion is larger than an area of the first heat-fusing portion.

According to aspect 12, this makes it more likely to tear the absorbent article along the lateral inner end of the joining portion after use while making the joining of the joining region stronger, compared with the case where the area of the joining portion is equal to or less than the area of the first heat-fusing portion.

Aspect 13 is the underpants-shaped absorbent article according to any one of the aspects 1 to 12, wherein in at least one of the waist portions, a plurality of elastic members that can stretch and contract in the lateral direction are arranged at intervals in the vertical direction, between the one-side sheet and the other-side sheet joined by a plurality of weld portions, the plurality of weld portions include a plurality of weld-portion pairs that are located on both sides of the elastic member in the vertical direction, when each of the elastic members contracts in the lateral direction, a position of the elastic member in the vertical direction relative to the one-side sheet and the other-side sheet is restricted by being sandwiched between the weld-portion pair, and an area of the joining portion is equal to or less than an area of the weld portion.

According to aspect 13, it is possible to reduce the risk of the joining region being excessively stiff compared with the case where the area of the joining portion is greater than the area of the weld portion.

Aspect 14 is the underpants-shaped absorbent article according to any one of the aspects 1 to 12, wherein in at least one of the waist portions, a plurality of elastic members that can stretch and contract in the lateral direction are arranged at intervals in the vertical direction, between the one-side sheet and the other-side sheet joined by a plurality of weld portions, the plurality of weld portions include a plurality of weld-portion pairs that are located on both sides of the elastic member in the vertical direction, when each of the elastic members contracts in the lateral direction, a position of the elastic member in the vertical direction relative to the one-side sheet and the other-side sheet is restricted by being sandwiched between the weld-portion pair, and an area of the joining portion is larger than an area of the weld portion.

According to aspect 14, this makes it more likely to tear the absorbent article along the lateral inner end of the joining portion after use while making the joining of the joining region stronger, compared with the case where the area of the joining portion is equal to or less than the area of the weld portion.

### Present embodiment

### Basic components of underpants-shaped disposable diapers

The following describes an underpants-shaped absorbent article according to the present embodiment by way of example of an underpants-shaped disposable diaper 1 (hereinafter also referred to as the "diaper 1"). However, the underpants-shaped absorbent article according to the present invention also includes a shorts-type sanitary napkin. Furthermore, the wearer of the underpants-shaped absorbent article is not limited to adults, but may be infants or living beings such as animals and the like.

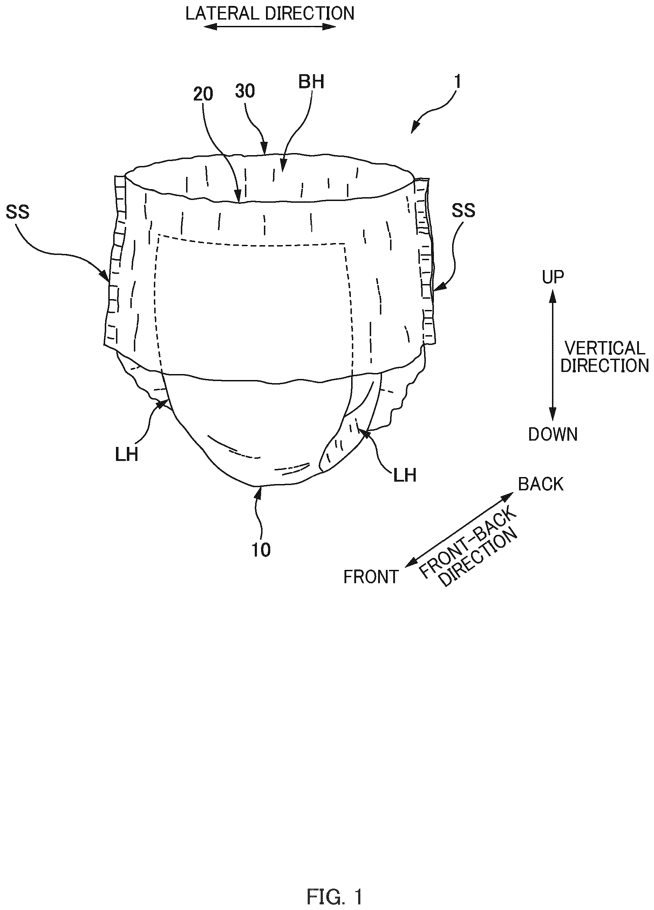

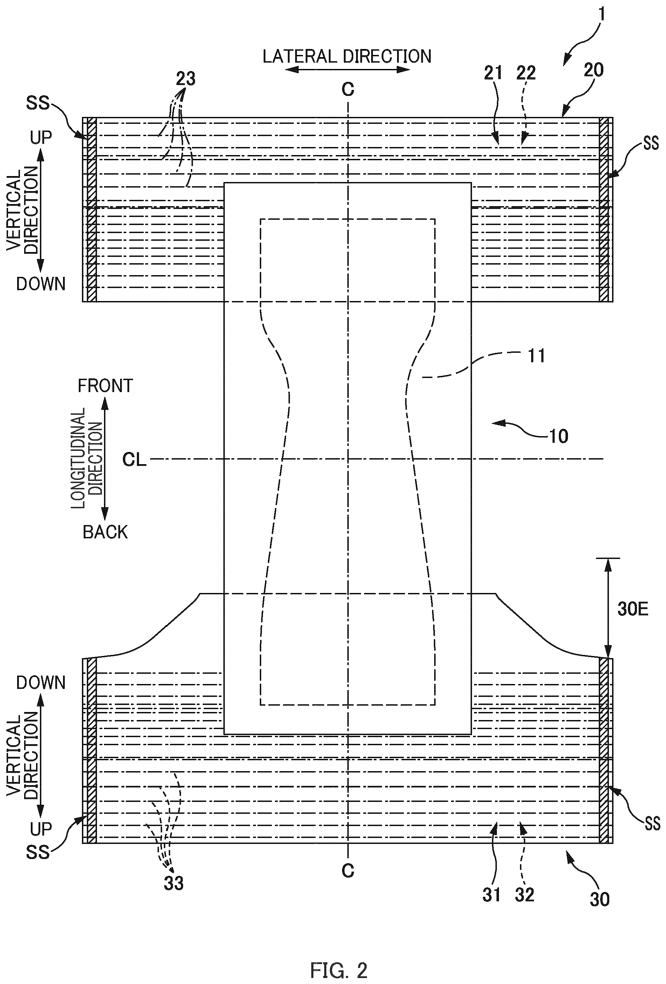

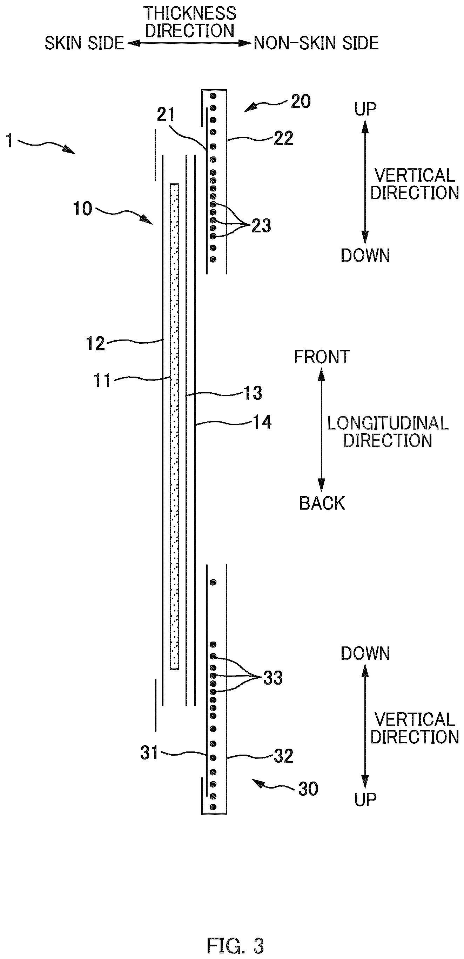

FIG. 1 is a schematic perspective view of the diaper 1. FIG. 2 is a schematic plan view of the diaper 1 in an unfolded and stretched state when viewed from the skin side of a wearer. FIG. 3 is a cross-sectional view taken along line A-A in FIG. 2. In FIG. 2, line C-C is the center line of the diaper 1 in the lateral direction (also referred to as a "lateral center C-C"), and line CL is the center line of the diaper 1 in the longitudinal direction (also referred to as a "longitudinal central CL").

The "unfolded state" of the diaper 1 refers to the state in which the diaper 1 in the underpants-shaped state of FIG. 1 is separated into a front waist portion 20 (described below) and a back waist portion 30 (described below) by detaching side joining portions SS (described below) on two sides in the lateral direction, and opening the diaper 1 in the vertical direction, thereby unfolding diaper 1 on a flat surface. In addition, the "stretched state" of the diaper 1 refers to the state in which the product (diaper 1) is stretched without wrinkles, specifically, the state of being stretched to the extent that the dimensions of constituent members of the diaper 1 (e.g., an absorbent main body 10, the front waist portion 20, etc.) match or are close to the dimensions of the members on their own. On the other hand, a "natural state" refers to the state when the diaper 1 or the front waist portion 20 is left on its own for a specific period of time. For example, the front waist portion 20 and the back waist portion 30 of the diaper 1 are pulled outward in the lateral direction to put the waist portions 20 and 30 in the "stretched state", the stretched state is maintained for 15 seconds, and then the diaper 1 is released and placed on a flat surface such as a desk. The state after five minutes of being laid flat on the flat surface is defined as the natural state.

The diaper 1 in the underpants-shaped state (FIG. 1) has a vertical direction, a lateral direction, and a front-back direction that intersect each other, and has a waist opening BH and a pair of leg openings LH. In the vertical direction, the side corresponding to the waist opening BH is the upper side, and the side corresponding to the wearer's crotch is the lower side. In the front-back direction, the side corresponding to the wearer's stomach is the front side, and the side corresponding to the wearer's back is the back side. Also, as shown in FIG. 3, the diaper 1 has a thickness direction in which members are stacked, and in the thickness direction, the side that comes into contact with the wearer is the skin side, and the opposite side is the non-skin side.

The diaper 1 includes an absorbent main body 10 and a pair of waist portions 20 and 30. The pair of waist portions 20 and 30 are joined to the non-skin-side surface of the absorbent main body 10 with an adhesive or the like. Out of the pair of waist portions 20, the one located on the front side will also be referred to as the front waist portion 20, and the one located on the back side will also be referred to as the back waist portion 30. The front waist portion 20 and the back waist portion 30 are joined by a pair of joining regions SS at both side end portions in the lateral direction.

As shown in FIG. 3, the absorbent main body 10 includes an absorbent core 11, a liquid-permeable top sheet 12 arranged on the skin side with respect to the absorbent core 11, a liquid-impermeable back sheet 13 arranged on the non-skin side with respect to the absorbent core 11, and an exterior sheet 14. The absorbent core 11 need to be any member that absorbs and retains excreted fluid, and examples thereof include those formed by molding a liquid-absorbent material such as pulp fibers or a superabsorbent polymer into a predetermined shape. The absorbent core 11 may also be covered with a liquid-permeable sheet made of tissue paper, nonwoven fabric, or the like. In addition, the absorbent main body 10 has leg elastic members, leak-proof wall portions including leak-proof-wall elastic members, and the like as appropriate, but these are omitted in FIG. 2 and the like.

In the present embodiment, the waist portions 20 and 30 are portions that overlap the joining regions SS with respect to the vertical direction, and the front waist portion 20 and the back waist portion 30 are portions that are rectangular in a plan view. An extension portion 30E having a substantially trapezoidal shape is provided on the crotch side of the back waist portion 30. The extension portion 30E can cover the buttocks of the wearer. In the following description, the back waist portion 30 and the extension portion 30E will be collectively referred to as the back exterior portions 30 and 30E. However, there is no limitation to this, and for example, the extension portion 30E need not be provided on the back side, or a portion extending from the front waist portion 20 toward the crotch may be provided.

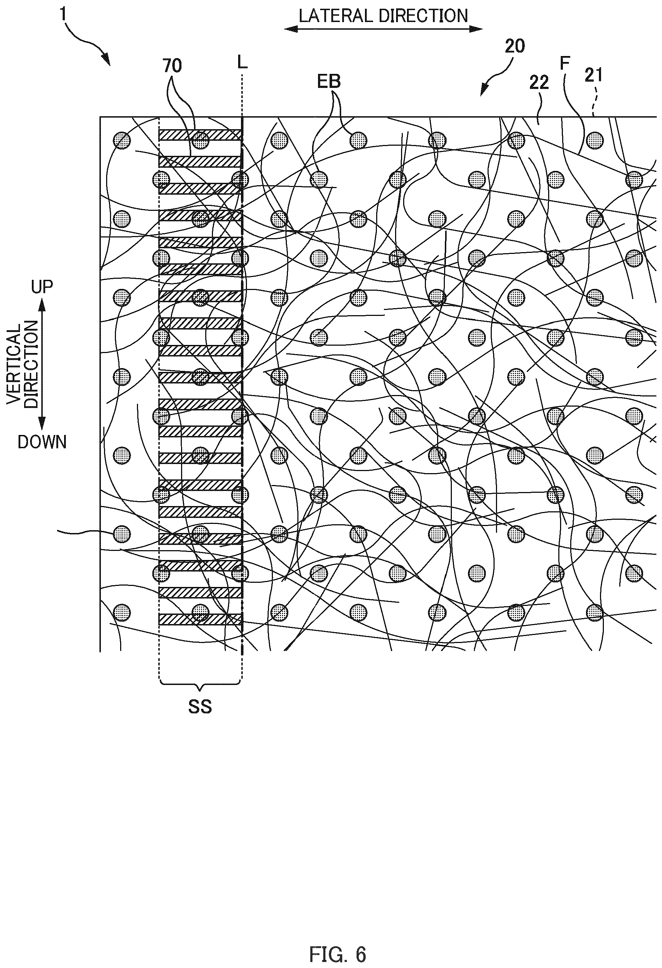

The front waist portion 20 includes a skin-side sheet (one-side sheet) 21 and a non-skin-side sheet (other-side sheet 22). In the present embodiment, spunbond nonwoven fabric is used for both of the skin-side sheet 21 and the non-skin-side sheet 22. However, the present invention is not limited to this, and other types of nonwoven fabric, such as SMS (spunbond/meltblown/spunbond) nonwoven fabric, meltblown nonwoven fabric, or the like may also be used. In this example, as the constituent fibers (heat-fusible fiber) F (see FIG. 6) of the nonwoven fabric, there are used single fibers made of polypropylene (PP), a typical example of a thermoplastic resin. But the present invention is not limited to this. For example, single fibers made of other thermoplastic resins such as polyethylene (PE) may be used, or further composite fibers having a sheath-core structure made of PE and PP or the like may be used. In addition, the following configuration is acceptable: either one of the sheets, the skin-side sheet 21 and the non-skin-side sheet 22 (e.g., the skin-side sheet 21) is made of spsunbond nonwoven fabric, and the other sheet (e.g., the non-skin-side sheet 22) is made of a flexible sheet member other than spunbond nonwoven fabric. In each of the skin-side sheet 21 and the non-skin-side sheet 22, a plurality of dot-shaped (circular) heat-fusing portions EB for fusing together heat-fusible fibers F made of thermoplastic resin and the like are provided in a dispersed manner (see FIG. 6 and the like). By providing the heat-fusing portions EB in the skin-side sheet 21 (non-skin-side sheet 22), the heat-fusible fibers F can be maintained in a sheet form. Furthermore, the heat-fusing portions EB do not join the skin-side sheet 21 and the non-skin-side sheet 22.

The back waist portion 30, like the front waist portion 20, includes a skin-side sheet (one-side sheet) 31 and a non-skin-side sheet (other-side sheet) 32. In the present embodiment, spunbond nonwoven fabric is used for both of the skin-side sheet 31 and the non-skin-side sheet 32. However, the present invention is not limited to this, and other types of nonwoven fabric, such as SMS (spunbond/meltblown/spunbond) nonwoven fabric, or the like may also be used. In this example, as the constituent fibers of the nonwoven fabric, there are used single fibers made of polypropylene (PP), a typical example of a thermoplastic resin. But the present invention is not limited to this. For example, single fibers made of other thermoplastic resins such as polyethylene (PE) may be used, or further composite fibers having a sheath-core structure made of PE and PP or the like may be used. In addition, the following configuration is acceptable: either one of the sheets, the skin-side sheet 31 and the non-skin-side sheet 32 (e.g., the skin-side sheet 31) is made of spsunbond nonwoven fabric, and the other sheet **(e.g.,** the non-skin-side sheet 32) is made of a flexible sheet member other than spunbond nonwoven fabric. In each of the skin-side sheet 31 and the non-skin-side sheet 32, a plurality of dot-shaped (circular) heat-fusing portions EB for fusing together heat-fusible fibers F made of thermoplastic resin and the like are provided in a dispersed manner. By providing the heat-fusing portions EB in the skin-side sheet 31 (non-skin-side sheet 32), the heat-fusible fibers F can be held in a sheet form. In addition, the heat-fusing portions EB do not join the skin-side sheet 31 and the non-skin-side sheet 32.

Although the basic configuration of the diaper 1 has been described above, the above-described configuration of the diaper 1 is merely an example, and the present invention is not limited to this. For example, in the diaper 1 of the present embodiment, the front waist portion 20 and the back exterior portions 30 and 30E are formed from separate members, and the non-skin-side surface of the absorbent main body 10 is exposed in the crotch portion. However, there is no limitation to this, and, for example, the diaper may include an exterior member constituted by three members including the front waist portion 20, the back exterior portions 30 and 30E, and a crotch portion connecting them to each other, or the diaper may include an exterior member constituted by a single continuous member extending from the front waist portion 20 to the back waist portion 30.

### Waist portion

The specific configurations of the front waist portion 20 and the back waist portion 30 will be described. As described above, the front waist portion 20 and the back waist portion 30 of the present embodiment have substantially the same configuration. Therefore, when the contents described below is the same for both the front waist portion 20 and the back waist portion 30, only the front waist portion 20 will be described as a representative for both. Regarding the back waist portion 30, the reference signs of its components and the like corresponding to those of the front waist portion 20 will be indicated in parentheses as necessary, and specific explanations will be omitted.

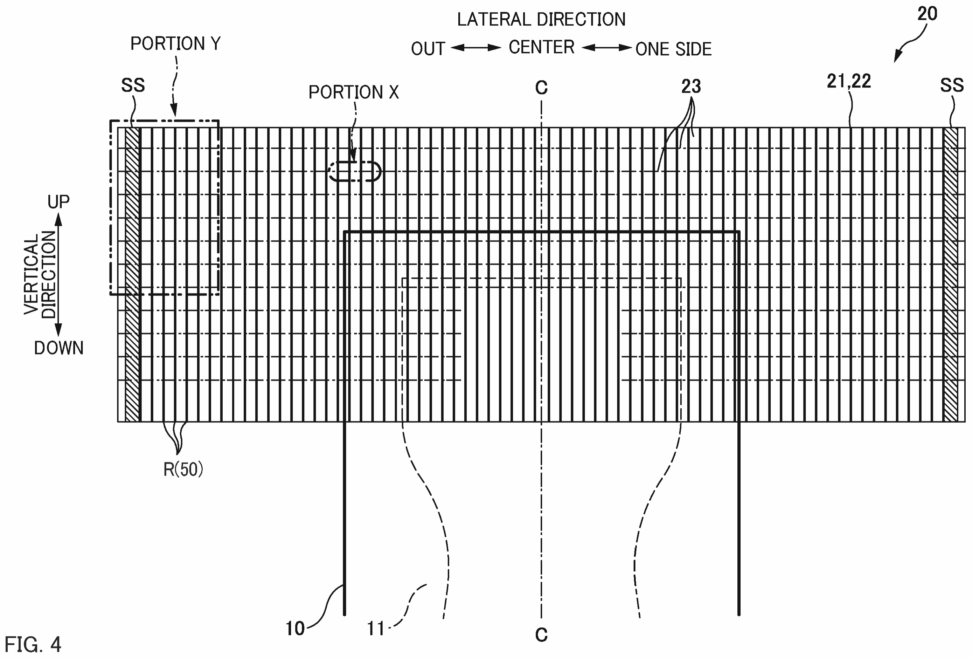

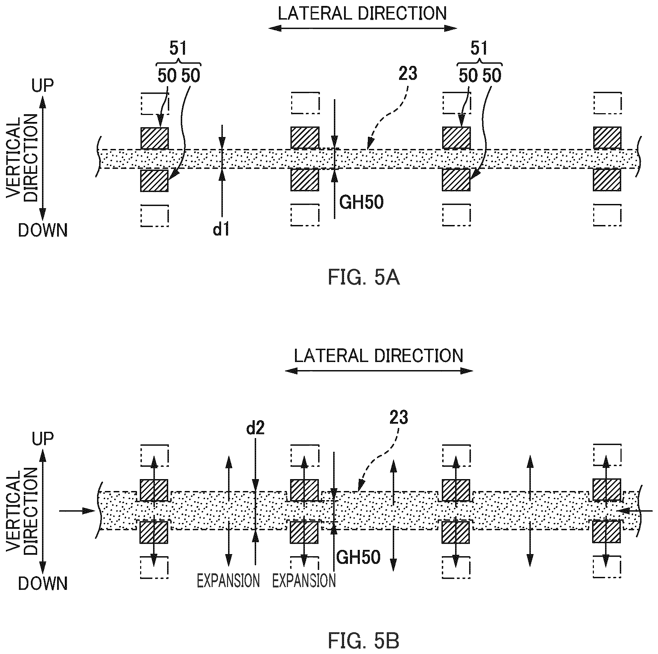

FIG. 4 is an illustrative diagram of weld portion rows Ra, Rb, and Rc provided in the front waist portion 20. FIGS. 5A and 5B are illustrative diagrams of attachment of the waist elastic members 23. In FIG. 4, the front waist portion 20 is shown in a state of being stretched in the lateral direction.

### Restriction of position of waist elastic members

The front waist portion 20 (30) of the present embodiment includes a plurality of weld portion rows R in each of which weld portions 50, 50,... are arranged side by side in the vertical direction. The weld portion rows R are arranged at intervals in the lateral direction. In FIG. 4, the weld-portion rows R are each depicted as a strip of area extending in the vertical direction, however, the actual weld-portion rows R are each formed by multiple weld portions 50 being arranged at intervals in the vertical direction.

As shown in FIG. 4, in the front waist portion 20 (30), a plurality of waist elastic members (elastic members) 23 (33) that can stretch and contract in the lateral direction are arranged at intervals in the vertical direction, between the skin-side sheet 21 (31) and the non-skin-side sheet 22 (32) joined by the plurality of weld portions 50 (between a pair of sheets). The skin-side sheet 21 (31) and the non-skin-side sheet 22 (32) are intermittently joined together by the weld portions 50 that are arranged discretely in the vertical direction and the lateral direction. Therefore, the front waist portion 20 (30) has stretchability in the lateral direction. In other words, the weld portions 50, 50... not only have the function of joining a pair of opposing surfaces of the skin-side sheet 21 (31) and the non-skin-side sheet 22 (32) to each other, but also have the function of attaching the waist elastic members 23 (33) to the skin-side sheet 21 (31) and the non-skin-side sheet 22 (32). The method for forming the weld portions 50 can be, for example, a well-known welding method such as ultrasonic welding or heat welding.

The pair of sheets joined by the weld portions 50 may be two separate sheets, such as the skin-side sheet 21 (31) and the non-skin-side sheet 22 (32) in the present embodiment, or may be two layers of a single sheet folded over onto itself. Furthermore, the weld portions 50 are not limited to joining only the pair of sheets sandwiching the waist elastic members 23 (33) therebetween, and may join three or more layers of sheets including a sheet overlaid on the pair of sheets.

As shown in FIG. 4, the plurality of weld portions 50 are provided at predetermined intervals in the vertical direction and in the lateral direction, and this makes it possible to suppress deterioration of texture of the diaper 1 when being worn while maintaining the flexibility of the front waist portion 20 (30). Assuming that a line-shaped weld portion extending continuously in the vertical direction or in the lateral direction is formed. In a portion where the weld portion is formed, nonwoven fabric becomes stiff, and, it is more likely to suppress deformation of nonwoven fabric in a direction in which the line-shaped weld portion extends. In contrast, the weld-portion row R in which the small welded portions 50 are dispersed as shown in FIG. 4 makes it possible to provide a flexible sheet member which makes a user (wearer) of diaper 1 less likely to feel curing of nonwoven fabric and makes it less likely to inhibit deformation of nonwoven fabric in the vertical direction and in the lateral direction.

In the front waist portion 20 (30), of the plurality of weld portions 50, 50,... included in the weld-portion row R, the waist elastic member 23 (33) is sandwiched above and below between two the weld portions 50 and 50 adjacent in the vertical direction, thereby attaching the waist elastic member 23 (33) to the front waist portion E 20 (30). That is, the pair of weld portions 50 and 50 arranged on both vertical sides of the waist elastic member 23 form a weld-portion pair 51, and the waist elastic member 23 (33) is attached by the weld-portion pair 51. FIGS. 5A and 5B are illustrative diagrams of attachment of the waist elastic member 23, and are schematic enlarged views of portion X in FIG. **4****.**

As shown in FIG. 5A, a pair of the weld portions 50 and 50 that make up the weld-portion pair 51 are spaced apart by a gap GH50 in the vertical direction. The size of the gap GH50 is set the same as or slightly larger than an outer diameter d1 (GH50 ≥ d1) of the waist elastic member 23 in the state where the front waist portion 20 is fully stretched in the lateral direction.

However, the waist elastic member 23 becomes thicker as it contracts in the lateral direction. Therefore, as shown in FIG. 5B, while the waist elastic member 23 is contracted in the lateral direction (i.e., in the natural state of the front waist portion 20), the gap GH50 of the weld portion pair 51 is smaller than a maximum outer diameter d2 (GH50 < d2) of the waist elastic member 23. In this way, the laterally contracted waist elastic member 23 is sandwiched by the weld portion pair 51 (i.e., expansion in the vertical direction is restricted). Therefore, the position (movement), in the lateral direction and the vertical direction, of the waist elastic member 23 relative to the skin-side sheet 21 and the non-skin-side sheet 22 is restricted.

Therefore, even without using an adhesive to fix the waist elastic member 23 to the skin-side sheet 21 and the non-skin-side sheet 22, positional shifting of the waist elastic member 23 can be suppressed, and the stretchability of the front waist portion 20 can be maintained. In other words, by utilizing the weld portions 50, the waist elastic member 23 can be attached to the skin-side sheet 21 and the non-skin-side sheet 22 without using an adhesive or with a reduced amount of adhesive. Therefore, it is possible to suppress a decrease in the flexibility of the front waist portion 20 (stretchable sheet 40) caused by hardening of an adhesive. Also, it is possible to suppress deterioration of the elastic properties of the waist elastic member 23 caused by hardening of an adhesive.

Also, in the front waist portion 20 (30), the waist elastic members 23 (33) are fixed to the skin-side sheet 21 (31) and the non-skin-side sheet 22 (32) in the joining regions SS. Specifically, during the manufacture of the diaper 1, the joining regions SS are formed while the waist elastic members 23 are stretched and sandwiched between the skin-side sheet 21 and the non-skin-side sheet 22, and thus the end portions of the waist elastic members 23 are fixed to the joining regions SS. Therefore, even if the front waist portion 20 (30) is stretched a large amount when putting on the diaper 1, the waist elastic members 23 (33) can be prevented from coming out from between the weld portion pairs 51, and the stretchability of the front waist portion 20 (30) is maintained.

Furthermore, the relationship between the gap GH50 of the weld portion pair 51 and the outer diameters d1 and d2 of the waist elastic member 23 is not limited to the above description. For example, the waist elastic members 23 may partially overlap the weld portions 50 as long as the waist elastic member 23 is not cut. In this case as well, the laterally contracted waist elastic member 23 is sandwiched by the weld portion pair 51, and the position, in the lateral direction and the vertical direction, of the waist elastic member 23 relative to the skin-side sheet 21 and the non-skin-side sheet 22 is restricted.

Furthermore, there is no limitation to a configuration in which the positions of all of the waist elastic members 23 (elastic members) arranged in the front waist portion 20 are restricted by being sandwiched by the weld portion pairs 51. It is sufficient that the positions of at least some of the waist elastic members 23 are restricted by the weld portion pairs 51. Other waist elastic members 23 may be fixed to the skin-side sheet 21 and the non-skin-side sheet 22 with an adhesive.

The pair of waist portions 20 and 30 are joined to each other by the pair of joining regions SS at side end portions in the lateral direction. In FIG. 4, the joining regions SS are each shown as a strip-shaped area extending in the vertical direction, however, the actual joining region SS is a region formed by the plurality of joining portions 70 lined up side-by-side at intervals in the vertical direction, as shown in FIG. 6, and is a region surrounded by straight lines extending along the vertical direction and connecting the lateral ends of the joining portions 70 adjacent in the vertical direction. The joining regions SS of the diaper 1 each extend continuously from the upper end to the lower end of a portion where the front waist portion 20 and the back waist portion 30 overlap when viewed in the front-back direction. FIG. 6 is a schematic enlarged view of a portion Y in FIG. 4. The joining portion 70 of the present embodiment has substantially a rectangular shape, and examples of joining methods include thermal welding, ultrasonic welding, and adhesive.

### Cutting waist portion after use

When removing an underpants-shaped absorbent article from the wearer's body after use, it is common to tear (cut) the one-side waist portion at a joining region (joining portion) to open the waist opening. FIG. 7A is a diagram illustrating the configuration of a conventional underpants-shaped absorbent article 2. FIG. 7B is a diagram illustrating a state of tearing the conventional underpants-shaped absorbent article 2. In the conventional underpants-shaped absorbent article 2, components having the same configuration as that of the diaper 1 of the present embodiment are denoted by the same reference numerals.

The underpants-shaped absorbent article 2 shown in FIGS. 7A and 7B is an example of a conventional underpants-shaped absorbent article. As shown in FIG. 7A, the underpants-shaped absorbent article 2 is joined in the both side end portions of the waist portions 20 and 30 by a plurality of joining portions 70 (joining region SS). The joining portions 70 of the underpants-shaped absorbent article 2 are semicircular weld portions, which are arranged intermittently in the vertical direction. This semicircular joining portions 70 are firmly joined in order not to separate the waist portions 20 and 30 when worn. When pulling the front waist portion 20 (one waist portion) at the joining portions 70 in an attempt to tear it off in order to remove the underpants-shaped absorbent article 2 from the wearer's body after use, the portions of the front waist portion 20 that are fixed to the back waist portion 30 (the other waist portion) by the joining portions 70 tend to remain fixed to the back waist portion 30, as shown in FIG. 7B. Therefore, there are a risk that the front waist portion 20 that has been torn off becomes in a state where the joining portions 70 are cut out (FIG. 7B), and a risk that the front waist portion 20 is torn in an unintended direction near the joining portion 70. In addition, the action of tearing off the front waist portion 20 along the contour of the joining portions 70 requires a force for strongly pulling the front waist portion 20. On the other hand, if the joining portion 70 is provided continuously from the upper end to the lower end of the front waist portion 20 in order to make it easier to cut the front waist portion 20 with the joining portion 70, then both side end portions of the waist portions 20 and 30 may become stiff, causing a risk that the wearer have a stiff and uncomfortable feeling.

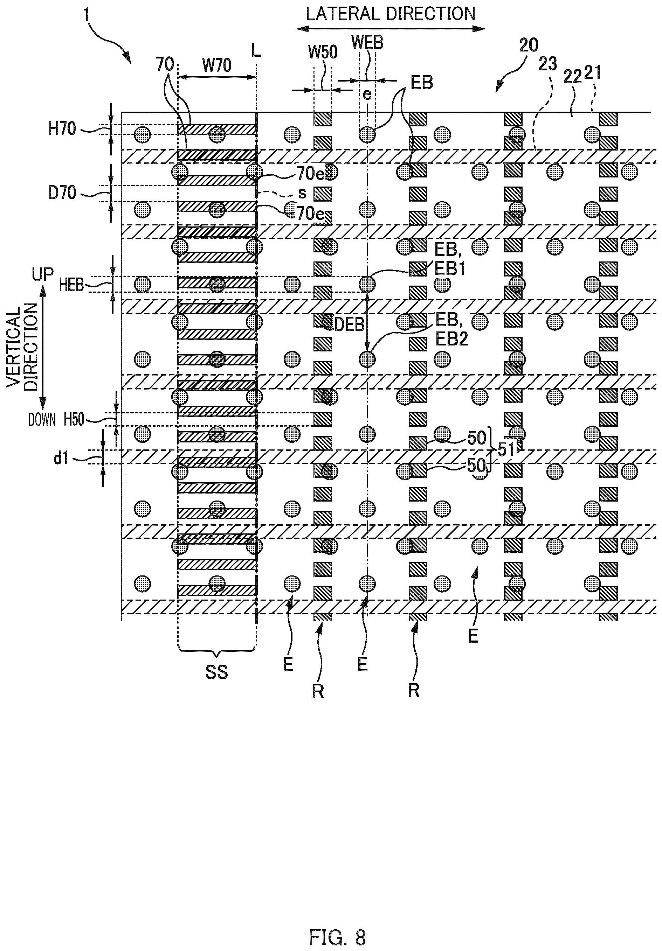

In contrast, in the diaper 1, it is easier to cut it along the inner ends of the joining portions 70 while reducing the risk of the joining regions SS becoming excessively stiff. FIG. 8 is a diagram for explaining the schematic enlarged view of. In FIG. 8, the heat-fusible fibers F in FIG. 6 are omitted, and the folded-back portion of the non-skin-side sheet 22 in the front waist portion 20 and the like are also omitted. In FIGS. 6, 8 and the like, the explanation will be given for the heat-fusing portions EB of the skin-side sheet 21, etc., and the same applies to the heat-fusing portions EB of the non-skin-side sheet 22.

As described above, the diaper 1 has a plurality of the joining portions 70 that are in the joining region SS and are aligned with the vertical direction. The front waist portion 20 (30) includes the skin-side sheet 21 and the non-skin-side sheet 22. The skin-side sheet 21 and the non-skin-side sheet 22 each have a plurality of the heat-fusing portions EB in which heat-fusible fibers F are joined together. These heat-fusing portions EB do not join the skin-side sheet 21 and the non-skin-side sheet 22. Of the plurality of heat-fusing portions EB, a group of the heat-fusing portions that are adjacent in the vertical direction in the stretched state and has the smallest central position e in the lateral direction in the stretched state forms the heat-fusing-portion row R. The plurality of heat-fusing portions EB in the present embodiment are arranged in a staggered pattern. Among the plurality of heat-fusing portions EB, a group of the heat-fusing portions EB whose lateral center e are located at the same position have the shortest central distance in the lateral direction. Such heat-fusing portions EB are ones whose lateral center e is the same, and these heat-fusing portions EB form a heat-fusing-portion row E. The heat-fusing-portion row E has a first heat-fusing portion EB1 and a second heat-fusing portion EB2, which are located inside the joining portions 70 in the lateral direction. In other words, the lateral center e of the first heat-fusing portion EB1 is also the lateral center e of the second heat-fusing portion EB2.

In this diaper 1, the gap D70 in the vertical direction between the joining portions 70 is wider than the length H70 of the joining portion 70 in the vertical direction, and the gap D70 in the vertical direction between the joining portions 70 is narrower than the gap DEB between the first heat-fusing portion EB1 and the second heat-fusing portion EB2 (H70 < D70 < DEB). In the diaper 1, the lateral center e of the first heat-fusing portion EB1 is the same as the lateral center e of the second heat-fusing portion EB2, and the first heat-fusing portion EB1 and the second heat-fusing portion EB2 are lined up in the vertical direction. Accordingly, the gap DEB between the first heat-fusing portion EB1 and the second heat-fusing portion EB2 is also the gap in the vertical direction between the first heat-fusing portion EB1 and the second heat-fusing portion EB2.

As shown in FIG. 8, in the joining regions SS of the diaper 1, the plurality of joining portions 70 of approximately the same size and shape are arranged with equal gaps (D70) in the vertical direction. The plurality of heat-fusing portions DB are approximately the same size and shape, and are arranged with equal gaps in a staggered pattern.

This makes it easier to tear along the lateral inner ends 70e of the joining portions 70 after use while reducing the risk of the joining region SS becoming excessively stiff, compared with the case where the gap D70 in the vertical direction between the joining portions 70 is wider than the length H70 of the joining portion 70 in the vertical direction and where the gap D70 in the vertical direction between the joining portions 70 is not narrower than the gap DEB between the first heat-fusing portion EB1 and the second heat-fusing portion EB2.

In general, the stiffness of the joining portion 70 is higher than where the joining portion 70 is not provided. Therefore, since the wider the joining portions 70 are formed, the stiffer both side end portions of the diaper 1 become, it is possible to reduce the risk of the joining region SS becoming excessively stiff in the diaper 1 by setting the gap D70 in the vertical direction between the joining portions 70 to be wider than the length H70 of the joining portion 70 in the vertical direction.

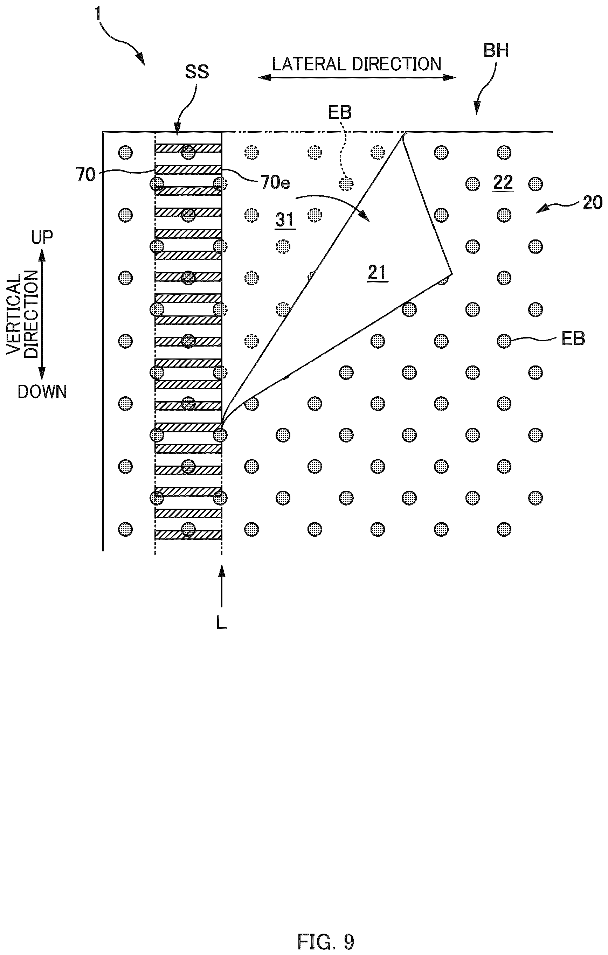

In addition, since the gap D70 in the vertical direction between the joining portions is narrower than the gap DEB in the vertical direction between the first heat-fusing portion EB1 and the second heat-fusing portion EB2, this makes it easier to tear at a cutting line L that extends along the lateral inner ends 70e of the joining portions. FIG. 9 is a diagram illustrating how the diaper 1 is torn.

As shown in FIG. 8, in the diaper 1, the cutting line L is a virtual line connecting, in the vertical direction, the lateral inner ends 70e of the joining portions 70 and straight lines s that extend along the vertical direction and connect the upper end 70e and lower end 70e of the joining portions 70 adjacent in the vertical direction. The straight line s is the shortest distance between the upper end 70e and the lower end 70e. In FIGS. 6 and 8, for convenience, the cutting line L is indicated by a double-dotted thick chain line. Within the cutting line L, the lateral inner ends 70e of the joining portions 70 are portions where a difference in stiffness occurs due to the joining portions 70, and this makes it easier to cut along the lateral inner ends of the joining portions 70. On the other hand, in the straight-lines s of the cutting line L, the difference in stiffness is small, and this makes it easier to cut them in an unintended direction. However, by making the gap D70 in the vertical direction between the joining portions 70 narrower than the gap DEB between the first heat-fusing portion EB1 and the second heat-fusing portion EB2, it makes it easier to apply the cutting force along the lateral inner ends 70e of the joining portions 70 directly to the cutting line L. This makes it easier to cut the front waist portion 20 along the cutting line L. Therefore, the cutting line L becomes a perforation-like cutting line, and it is possible to cut the front waist portion 20 efficiently along the cutting line L with a small force, as shown in FIG. 9. Also, it is possible to reduce the risk of tearing the front waist portion 20 in an unintended direction.

In addition, in the cutting line L, the straight lines s are each provided continuing to the lateral inner ends 70e of the joining portions 70 that are adjacent thereto in the vertical direction, and therefore the cutting line L can be made into a continuous shape, making it easier to cut the front waist portion 20 efficiently along the cutting line L. Furthermore, in the cutting line L, it is more preferable that the lateral inner ends 70e of the joining portions 70 and the straight lines s are a single straight line without bending. This allows the cutting line L to be straight, making it easier to cut the front waist portion 20 efficiently with a smaller force. In addition, although the cutting line L of the diaper 1 of the present embodiment extends in the vertical direction (parallel to the vertical direction), the present invention is not limited to this. The cutting line L may be inclined relative to the vertical direction, and may have a jagged or wavy shape with bends or curves. The shape of the cutting line L can be changed depending on the shape and arrangement of the joining portions 70. By changing the shape and arrangement of the joining portions 70 and thereby changing the shape of the cutting line L, it is possible to adjust the joining strength of the joining portions 70 and to adjust the texture given to the wearer according to the stiffness of the joining portions 70.

For the joining portions 70 in the joining region SS, it is preferable that the lateral length W70 of the joining portion 70 is longer than the vertical length H70 of the joining portion 70 (H70 < W70). This makes it easier to tear the front waist portion 20 along the inner ends 70e of the joining portions 70 after use while making it easier to increase the joining strength in the joining portions 70 compared with the case where the vertical length H70 of the joining portion 70 is shorter than the lateral length W70 of the joining portion 70.

It is preferable that the vertical gap D70 between of the joining portions is narrower than the lateral length W70 of the joining portion 70 (D70 < W70). This makes it easier to tear the front waist portion 20 along the lateral inner ends 70e of the joining portions 70 after use while making it easier to increase the joining strength in the joining portion 70 compared with the case where the vertical gap D70 between the joining portions 70 is wider than the lateral length W70 of the joining portion 70.

It is also preferable that the vertical length H70 of the joining portion is shorter than the vertical length HEB of the first heat-fusing portion EB1 (heat-fusing portion EB) (H70 < HEB). The longer the vertical length H70 of the joining portion 70, the higher the stiffness of the joining region SS. Accordingly, by making the vertical length H70 of the joining portion 70 shorter than the vertical length HEB of the first heat-fusing portion EB1, it is possible to further reduce the risk of the joining region SS becoming excessively stiff compared with the case where the vertical length H70 of the joining portion 70 is longer than the vertical length HEB of the first heat-fusing portion EB1.

In the front waist portion 20 of the diaper 1, between the skin-side sheet 21 and the non-skin-side sheet 22, the plurality of waist elastic members 23 that can stretch and contract in the lateral direction are arranged at intervals in the vertical direction. In this diaper 1, it is preferable that the vertical length H70 of the joining portion 70 is shorter than the thickness d2 of the elastic member in the natural state (see FIG. 5B). Also, it is more preferable that the vertical length H70 of the joining portion 70 is shorter than the thickness d1 of the elastic member in the stretched state.

During the manufacture of the diaper 1, the joining portions 70 (joining region SS) are formed with the waist elastic members 23 in the stretched state being sandwiched between the skin-side sheet 21 and the non-skin-side sheet 22. At this time, the end portions of the waist elastic members 23 are fixed to the joining region SS, but if the joining portions 70 are formed by welding at a position overlapping the waist elastic member 23 when the diaper 1 is viewed from the thickness direction, there is a risk that the waist elastic members 23 are cut. In contrast, by making the vertical length H70 of the joining portion 70 shorter than the thickness d2 of the elastic member in the natural state, and more preferably, by making the vertical length H70 of the joining portion 70 shorter than the thickness d1 of the elastic member in the stretched state, it is possible to reduce the risk of the joining portion 70 cutting the waist elastic member 23. In the case where the vertical length H70 of the joining portion 70 is made shorter than the thicknesses d2 and d1 of the elastic member, for example, it is possible to make it less likely for the waist elastic member 23 and the joining portion 70 to overlap when forming the joining portion 70. Further, even if the waist elastic member 23 and the joining portion 70 do overlap, the risk of the waist elastic member 23 being completely cut can be reduced by simply removing a portion of the waist elastic member 23.

In particular, in the case where the waist elastic members 23 are attached to the skin-side sheet 21 and the non-skin-side sheet 22 without using adhesive or by using a reduced amount of adhesive, as in diaper 1, it becomes important to fix the waist elastic member 23 with the joining region SS. In such a case, in order to reduce the risk of cutting of the waist elastic members 23, it is preferable to make the vertical length H70 of the joining portion 70 shorter than the thicknesses d2 and d1 of the elastic member.

It is preferable that, in the front waist portion 20, the plurality of waist elastic members 23 are located separated apart from each other in the vertical direction. This can reduce the risk the waist elastic member 23 excessively tighten around the wearer's body when worn, while also reducing the risk that the plurality of waist elastic members 23 interfere with cutting the front waist portion 20 when cutting it after use.

Furthermore, as described above, when each of the waist elastic members 23 contracts in the lateral direction, the position of the waist elastic member 23 in the vertical direction relative to the skin-side sheet 21 and the non-skin-side sheet 22 is restricted by being sandwiched between the weld-portion pair 51 (a pair of the weld portions 50 and 50), it is preferable that the lateral length W70 of the joining portion 70 is longer than the lateral length W50 of the weld portion 50 (W70 > W50). The joining region SS of the diaper 1 is required to be more firmly attached in order to maintain the underpants-shaped state. Therefore, by making the lateral length W70 of the joining portion 70 longer than the lateral length W50 of the weld portion 50, the joining strength of the joining region SS can be increased compared with the case where the lateral length W70 of the joining portion 70 is shorter than the lateral length W50 of the weld portion 50.

For the weld portion 50, it is preferable that the area S70 of the joining portion 70 is larger than the area S50 of the weld portion 50 (S70 > S50). Because the area 70 of one joining portion 70 in the joining region SS is larger than the area S50 of one weld portion 50, this makes it more likely to tear the diaper 1 at the lateral inner ends 70e of the joining portions 70 after use while making the joining of the joining region SS stronger, compared with the case where the area S70 of the joining portion 70 is equal to or less than the area S50 of the weld portion 50.

The area S70 of the joining portion 70 may be equal to or less than the area S50 of the weld portion 50 (S70 ≤ S50). This can reduce the risk of the joining region SS being excessively stiff compared with the case where the area S70 of the joining portion 70 is greater than the area S50 of the weld portion 50.

It is also preferable that the gap DEB between the first heat-fusing portion EB1 and the second heat-fusing portion is greater than twice the sum of the vertical length H70 of the joining portion 70 and the vertical gap D70 between the joining portions 70 (DEB > (H70 + D70) × 2). This makes it easier to tear the front waist portion 20 along the lateral inner ends 70e of the joining portions 70 after use compared with the case where the gap DEB between the first heat-fusing portion EB1 and the second heat-fusing portion EB2 is narrower than twice the sum of the vertical length H70 of the joining portion 70 and the vertical gap D70 between the joining portions 70.

It is preferable that the lateral length W70 of the joining portion 70 is longer than the lateral length WEB of the first heat-fusing portion EB1 (W70 > WEB). This allows the joining strength of the joining region SS to increase compared with the case where the lateral length W70 of the joining portion 70 is shorter than the lateral length WEB of the first heat-fusing portion EB1.

Also, it is preferable that the area S70 of one joining portion 70 in the joining region SS is larger than the area SEB of the first heat-fusing portion EB1 (S70 > SEB). This makes it easier to tear the front waist portion 20 along the lateral inner ends 70e of the joining portions 70 after use while making the joining of the joining region SS stronger compared with the case where the area S70 of the joining portion 70 is equal to or less than the area SEB of the first heat-fusing portion EB1.

The area S70 of the joining portion 70 may be equal to or less than the area SEB of the first heat-fusing portion EB1 (S70 ≤ SEB). This can reduce the risk of the joining region SS becoming excessively stiff compared with the case where the area S70 of the joining portion 70 is larger than the area SEB of the first heat-fusing portion EB1.

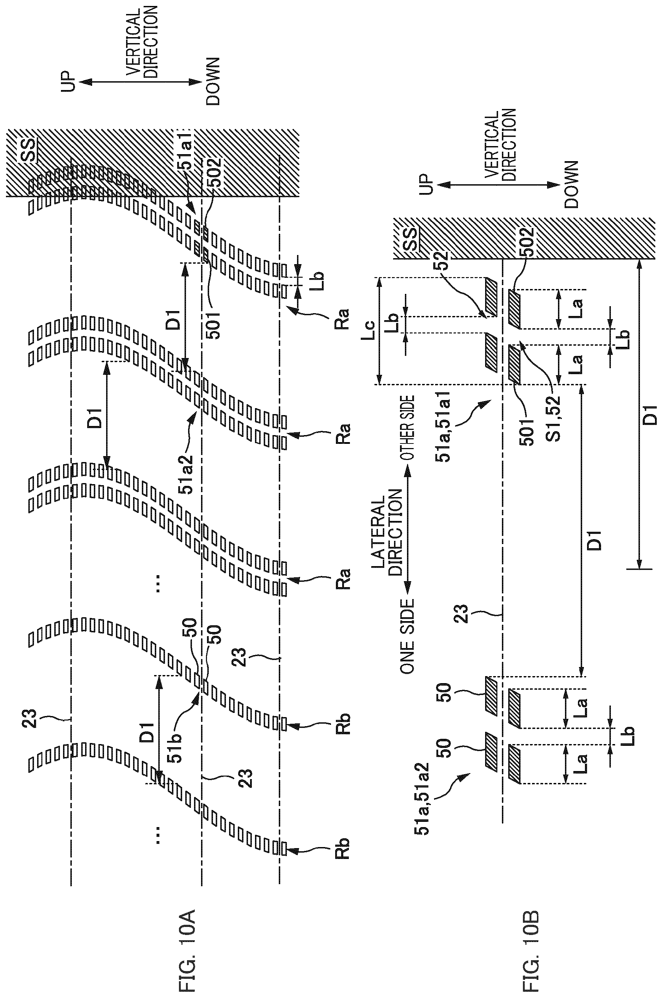

In the present embodiment (FIG. 4 and the like), each weld-portion row R is arranged in a straight line along the vertical direction, but the weld-portion rows R may also be arranged meandering in the lateral direction. Some of weld portions 50 constituting the weld-portion row R may each be located offset along the lateral direction (FIG. 10A). In addition, in the present embodiment, the plurality of weld-portion rows R are arranged at approximately equal intervals in the lateral direction, but the intervals in the lateral direction between the plurality of weld-portion rows R can be changed as desired. For example, in the front waist portion 20, the following configuration may be acceptable: the intervals in the lateral direction between a plurality of the weld-portion rows R at the ends in the lateral direction is reduced, and the intervals the lateral direction between a plurality of the weld-portion rows R in the center in the lateral direction is increased.

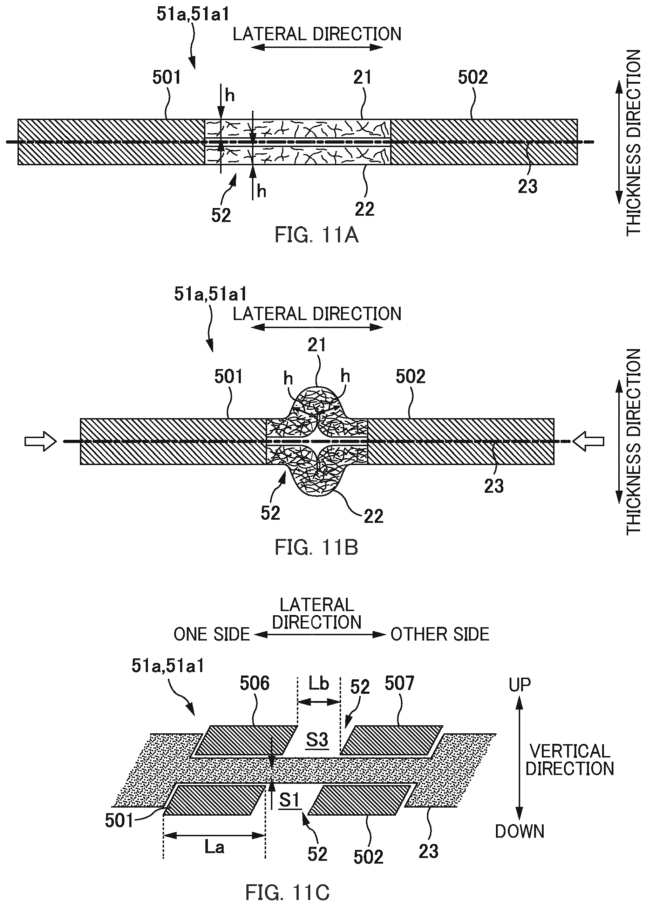

As shown in FIG. 10A, the front waist portion 20 (30) may have two types of the weld-portion row, an "interrupted-weld-portion row Ra" and a "normal-weld-portion row Rb". The following describes a modified example of the weld-portion rows R. In addition, with regard to the modified example of the underpants-shaped absorbent article, the same symbols will be given to configurations that are the same as those in the diaper 1 described above, and the description thereof will be omitted. FIG. 10A is a diagram illustrating the weld-portion rows Ra and Rb of the modified example, and FIG. 10B is a diagram illustrating an interrupted-weld-portion pair 51a. FIG. 11A is a diagram illustrating the interrupted-weld-portion pair 51a in the stretched state, and FIGS. 11B and 11C are diagrams illustrating the interrupted-weld-portion pair 51a in the contracted state.

In the front waist portion 20 of the modified example, the plurality of weld-portion rows Ra and Rb are lined up in the lateral direction with a predetermined gap D1. The gap D1 in the lateral direction between the weld-portion rows (Ra, Rb) can be, for example, about 4 to 14 times the length La of the weld portion 50 in the lateral direction. The plurality of weld-portion rows Ra, Rb are arranged with a relatively wide gap. Hereinafter, among the weld portions 50 belonging to the interrupted-weld-portion rows Ra, the weld-portion pair 51 between which the waist elastic member 23 are sandwiched is referred to as an "interrupted-weld-portion pair 51a," and among the weld portions 50 belonging to the normal-weld-portion row Rb, the weld-portion pair 51 between which the waist elastic member 23 are sandwiched is referred to as a "normal-weld-portion pair 51b".

As shown in FIG. 10A, in the normal-weld-portion row Rb, the weld portions 50 are lined up in a single row in the vertical direction. Thus, the normal-weld-portion pair 51b is made up of two weld portions 50 which are respectively located on both sides of the waist elastic member 23 in the vertical direction.

On the other hand, in the interrupted-weld-portion row Ra, a set of two weld portions 50 adjacent in the lateral direction at a close gap are lined up in the vertical direction. The interrupted-weld-portion row Ra is constituted by two of the weld-portion rows. Therefore, as shown in FIG. 10B, the interrupted-weld-portion pair 51a is made up of four weld portions 50, two on each side of the waist elastic member 23 in the vertical direction.

In FIG. 10B, the interrupted-weld-portion pair 51a located near the joining region SS is referred to as a "first interrupted-weld-portion pair 51a1 (first weld-portion pair)". The interrupted-weld-portion pair 51a located adjacent to the above on the one side in the lateral direction is referred to as a "second interrupted-weld-portion pair 51a2 (second weld-portion pair) ". The first interrupted-weld-portion pair 51a1 and the second interrupted-weld-portion pair 51a2 sandwich the same waist elastic member 23 (first elastic member) between them.

The first interrupted-weld-portion pair 51a1 has a first weld portion 501 and a second weld portion 502, which are located on the same side in the vertical direction (below in this example) with respect to the waist elastic member 23 and are located spaced from each other with a first gap S1 in the lateral direction.

Then, in a state where the front waist portion 20 is stretched in the lateral direction, the lateral length D1 (first length) from the lateral one-side end of the first interrupted-weld-portion pair 51a1 to the lateral other-side end of the second interrupted-weld-portion pair 51a2 is longer than the lateral length Lb of the first gap S1 (D1 > Lb).

In the following description, the length D1 is also referred to as a "weld-portion pair gap D1". Additionally, the lateral length Lb of the first gap S1 is the length from an end of the first weld portion 501 on the other side in the lateral direction (second weld portion 502 side) to an end of the second weld portion 502 on the one side in the lateral direction (first weld portion 501 side).

As described above, in the interrupted-weld-portion pair 51a, a portion of the weld portion 50 is missing on the one side of the waist elastic member 23in the vertical direction, and a non-weld-portion 52 is formed between the weld portions (501 and 502) located side-by-side in the lateral direction. The non-weld-portion 52 is an area of approximately the same length as the lateral length Lb of the first gap S1, and is a narrower area than the weld-portion pair gap D1. Therefore, when the waist elastic member 23 contracts (FIG. 11B), the two weld portions (501 and 502) located outside the non-weld-portion 52 on both sides approach each other, and thus portions of the skin-side sheet 21 and the non-skin-side sheet 22 (hereinafter also referred to as "portions of the sheets 21 and 22") located in the non-weld-portion 52 are sandwiched between these two weld portions (501 and 502). As a result, in the non-weld-portion 52, the density of the fibers constituting the portions of the sheets 21 and 22 increases.

By providing the non-weld-portion 52 that is a gap Lb narrower than the gap D1 of the weld-portion pair, like the interrupted-weld-portion pair 51a, when the waist elastic member 23 contracts, the portions of the sheets 21 and 22 in the non-weld-portion 52 can be sandwiched between the weld portions (501 and 502), thereby increasing the fiber density of the portions of the sheets 21 and 22 in the non-weld-portion 52. Specifically, Lb is preferably 2.54 mm or less, and more preferably about 0.5 **mm.** As a result, in the non-weld-portion 52, the dense fibers become more likely to be entangled with the waist elastic member 23, making it possible to restrict the movement of the waist elastic member 23. Therefore, it is possible to prevent the waist elastic member 23 from coming off from the interrupted-weld-portion pair 51a.

In particular, in the underpants-shaped diaper 1, the sheets 21 and 22 are welded relatively strongly in the process of forming the side joining portions SS during the manufacture of the diaper 1, and therefore the waist elastic member 23 may be cut. Furthermore, at the time of putting on the diaper 1, when the joining regions SS are held and the diaper 1 is pulled up, a strong pulling force acts on the joining regions SS, which may cause the waist elastic member 23 to break. Even if the waist elastic member 23 comes off from the joining region SS, the waist elastic member 23 contracting from the joining region SS is restricted by the interrupted-weld-portion pair 51a, making it possible to prevent the waist elastic member 23 from coming off from the interrupted-weld-portion pair 51a. Therefore, the decrease in stretchability of the front waist portion 20 can be suppressed.

In addition, in the interrupted-weld-portion pair 51a, the non-weld-portion 52 is provided in a part in the lateral direction. Therefore, compared with the case of providing the normal-weld-portion pair 51b with the same length Lc as the interrupted-weld-portion pair 51a, the area ratio of the weld portion 50 in the lateral direction can be reduced, and the flexibility of the front waist portion 20 can be secured. In addition, the non-weld-portion 52 is likely to be the base point of folding of the front waist portion 20 in the lateral direction. Therefore, the front waist portion 20 can easily fold in the lateral direction and easily fit around the wearer's waist.

As described above, by providing the interrupted-weld-portion pair 51a to the front waist portion 20, it is possible to prevent the waist elastic member 23 from coming off, and to suppress the decrease in stretchability, and to ensure the flexibility of the front waist portion 20. In other words, by using the weld-portion pair 51 to restrict the position of the waist elastic member 23 relative to the sheets 21 and 22, the amount of adhesive used can be reduced, thereby ensuring the flexibility of the front waist portion 20.

In addition, in the example of the front waist portion 20 in FIG. 10, the interrupted-weld-portion rows Ra with a laterally-meandering shape (interrupted-weld-portion pair 51a) are arranged near the joining region SS and near the side portions of the absorbent main body 10. However, the shape, number, and arrangement of the interrupted-weld-portion rows Ra are not particularly limited, as long as the front waist portion 20 has at least one interrupted-weld-portion pair 51a. In addition, in FIG. 10, the shape of the weld portion 50 is a parallelogram, but the shape of the weld portion 50 is not particularly limited. Furthermore, the shapes of the weld portions 50 belonging to each weld-portion row Ra, Rb may be the same or different.

In addition, all of the weld-portion rows provided in the front waist portion 20 may be interrupted-weld-portion rows Ra, but it is preferable that the weld-portion rows includes both of the normal-weld-portion row Rb consisting of one row and the interrupted-weld-portion row Ra. This makes it possible to prevent the waist elastic member 23 from coming off from the weld-portion pair 51 while retaining the flexibility from the front waist portion 20.

In the present embodiment, the weld portions 50 and the joining portions 70 are each rectangular in shape, and the weld portions 50 and the joining portions 70 are arranged along the vertical direction and the lateral direction, the heat-fusing portions EB are circular in shape, and the plurality of heat-fusing portions EB are arranged in a staggered pattern. But the present invention is not limited to this. The weld portion 50, the joining portion 70, and the heat-fusing portion EB can each be of any shape and can be arranged in any configuration.

The joining portion 70 is not limited to a rectangular shape, but may be any shape such as a circle, an ellipse, or a semicircle. However, it is preferable that the lateral inner ends 70e of the joining portions 70 is aligned in the vertical direction. The lateral inner ends 70e of the joining portions 70 are arranged along in the vertical direction, making it easier to cut the front waist portion 20 along the cutting line L after use.

Moreover, the shape of the heat-fusing portion EB is not limited to a circle, but may be any shape such as a rectangle or an ellipse. Furthermore, the arrangement of the heat-fusing portions EB is not limited to a staggered pattern. They may be arranged in a grid pattern with equal intervals, or does not have to be arranged in line along the vertical direction and the lateral direction, and does not have to be arranged with equal gaps.

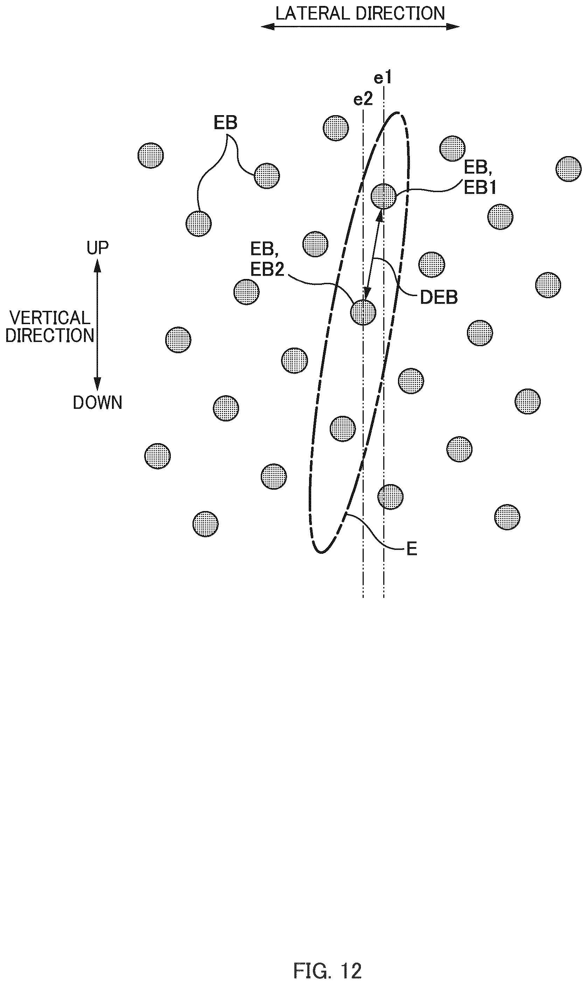

FIG. 12 is a diagram showing the modified example of the heat-fusing portions EB. The plurality of heat-fusing portions EB of the modified example shown in FIG. 12 are arranged in a staggered pattern in the stretched state, but the plurality of heat-fusing portions EB are arranged slightly offset from the vertical direction and the lateral direction. In such a case, the heat-fusing-portion row E and E are formed along with the vertical direction by the group of the heat-fusing portions which are adjacent to each other in the vertical direction and are surrounded by a double dotted line in FIG. 12, where the lateral central positions *e1* and e2 are the smallest. The heat-fusing-portion row E may have a second heat-fusing portion EB2 with the first heat-fusing portion EB1. In such a case, the gap DEB between the first heat-fusing portion EB1 and the second heat-fusing portion EB2 is the shortest distance DEB in a straight line connecting the outer end of the first heat-fusing portion EB1 and the outer end of the second heat-fusing portion EB2.

### Manufacturing method of diaper 1

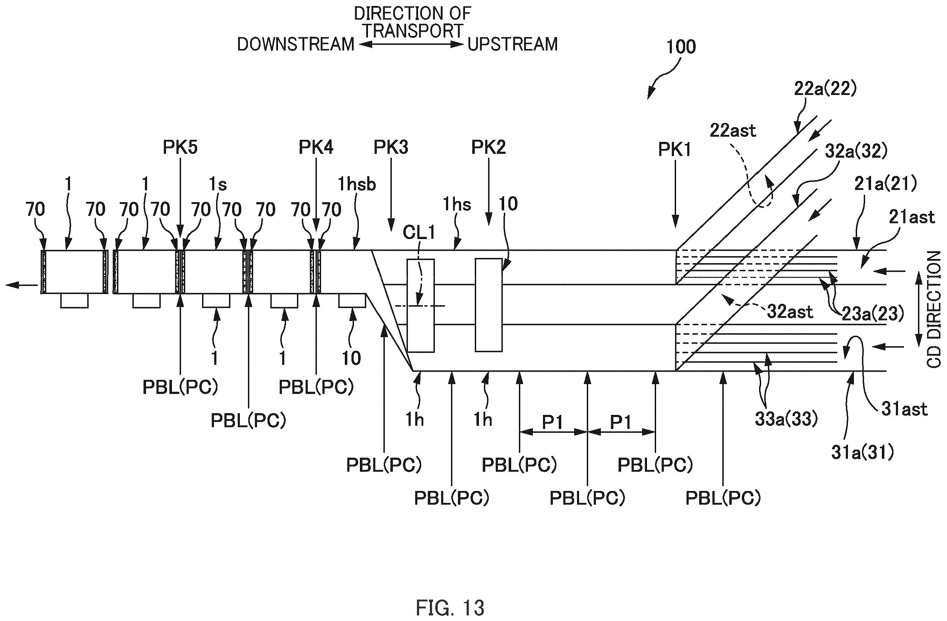

Next, a method for manufacturing the diaper 1 will be described. The diaper 1 is manufactured by the manufacturing line 100. FIG. 13 is a schematic plan view, with a partial perspective view, showing the process of manufacturing the diapers 1 in the manufacturing line 100.

In the manufacturing line 100, for example, two nonwoven fabric sheets 21 and 22 related to the front waist portion 20 (corresponding to the skin-side sheet 21 and the non-skin-side sheet 22) are transported in the form of the continuous sheets 21a and 22a connected in the direction of transport (corresponding to continuous bodies of each of the sheets). Similarly, two nonwoven fabric sheets 31 and 32 related to the back waist portion 30 (corresponding to the skin-side sheet 31, the non-skin-side sheet 32) are transported in the form of the continuous sheets 31a and 32a connected in the direction of transport (corresponding to continuous bodies of each of the sheets). Then, each time when a set of two continuous sheets 21a and 22a (31a and 32a) pass through a plurality of processing positions PK1 to PK5 determined in the direction of transport, a processing corresponding to each processing position PK1, PK2, ... is performed on the set of two continuous sheets 21a and 22a (31a and 32a).

In addition, when the direction perpendicular to both the thickness direction and the direction of transport of the continuous sheets 21a, 22a, 31a, 32a is defined as "CD direction", in this example, the sets of two continuous sheets 21a and 22a (31a and 32a), **i.e.,** two continuous sheets 21a and 22a related to the front waist portion 20 and two continuous sheets 31a and 32a related to the back waist portion 30, are transported side by side in the CD direction. However, the present invention is not limited to this.

In this example, the plurality of processing positions are determined as a first processing position PK1 to a fifth processing position PK5, arranged in this order from upstream to downstream in the direction of transport. The processing at each processing position PK1, PK2,... for the two continuous sheets 21a and 22a related to the front waist portion 20 and for the two continuous sheets 31a and 32a related to the back waist portion 30 are substantially the same.

Therefore, in the following, common content will be described without distinguishing between the front waist portion 20 and the back waist portion 30. For example, the description may be made by simply using the term "belt member 20 (30)" or simply using the term "two continuous sheets 21a and 22a (31a and 32a).". In such cases, the numeral immediately following the term indicating each component is the symbol for the component related to the front waist portion 20, and the numeral in parentheses following to it is the symbol for the component related to the back waist portion 30, such as "continuous sheets 21a and 22a (31a and 32a)," "elastic member 23 (33)," "the elastic-member continuous body 23a (33a)," and the like.

As shown in FIG. 13, the two continuous sheets 21a and 22a (31a and 32a) of each belt member 20 (30) are conveyed in a so-called lateral flow manner. That is, the two continuous sheets 21a and 22a (31a and 32a) are conveyed with the direction corresponding to the lateral direction of the diaper 1 conforming to the direction of transport. Therefore, for the two continuous sheets 21a and 22a (31a and 32a), the boundary position PBL between the adjacent diapers 1 and 1 in the lateral direction is virtually set at the product pitch P1 in the direction of transport. Then, at a fifth processing position PK5 located at the end of the manufacturing line, the two continuous sheets 21a and 22a (31a and 32a) are cut at a cutting target position PC, which is the boundary position PBL, thereby producing a single-cut diaper 1.

The two continuous sheets 21a and 22a (31a and 32a) of each belt member 20 (30) are transported by an appropriate transporting device (not shown) such as a belt conveyor or transport rollers. Therefore, unless otherwise specified, it is assumed that the two continuous sheets 21a and 22a (31a and 32a) are transported in the direction of transport by these transporting devices. Examples of belt conveyors include a normal belt conveyor having a rotating endless belt as a conveying surface, and a suction belt conveyor having a sucking function on the outer circumferential surface of the endless belt.

When manufacturing the diaper 1, as shown in FIG. 13, first, the two continuous sheets 21a and 22a (31a and 32a) relating to each belt member 20 (30) pass through the first processing position PK1. During this passage, the two continuous sheets 21a and 22a (31a and 32a) are overlapped with each other in the thickness direction. In addition, when overlapping the two continuous sheets 21a and 22a (31a and 32a) to each other, the elastic-member continuous bodies 23a, 23a... (33a, 33a...) that are continuous in the direction of transport are placed side-by-side in the CD direction between a pair of opposing surfaces 21ast and 22ast (31ast and 32ast) that face each other in the two continuous sheets 21a and 22a (31a and 32a), in a state where the elastic-member continuous bodies 23a, 23a... (33a, 33a...) stretch in the direction of transport at the above-described target stretch factor.

Also, simultaneously with or immediately after this overlapping, the above-mentioned weld portions 50, **50,...** are formed as the joining portions between the two continuous sheets 21a and 22a (31a and 32a), and thus a pair of opposing surfaces 21ast and 22ast (31ast and 32ast) of the two continuous sheets 21a and 22a (31a and 32a) are joined by the weld portions 50, 50,....

Here, in the manufacturing line 100, the lateral direction of the diaper 1 conforms to the direction of transport, and the vertical direction of the diaper 1 conforms to the CD direction. Therefore, the weld portions 50 are formed in pairs, on both sides of the elastic-member continuous bodies 23a (33a) in the CD direction. Specifically, the pair of weld portions 50 and 50 that are located side-by-side on respective side of the elastic-member continuous bodies 23a (33a) in the CD direction form the weld-portion pair 51. The plurality of such weld-portion pairs 51 are formed side-by-side in the direction of transport while spacing between the weld-portion pairs 51 that are adjacent in the direction of transport (see FIG. 4, and the like).

Such weld portions 50 can be formed using a heat sealing device or an ultrasonic welding device, for example. Note that a heat sealing device has a pair of rolls that are heated while rotating in the direction of transport, for example. One of the rolls is a heat embossing roll that has protrusion portions corresponding to the weld portions 50 on the outer circumferential surface, and the other roll is an anvil roll that receives the protrusion portions with a smooth outer circumferential surface. The ultrasonic welding device can also be used.

Next, as shown in FIG. 13, the two continuous sheets 21a and 22a related to the front waist portion 20 and the two continuous sheets 31a and 32a related to the back waist portion 30 all pass through the second processing position PK2. During this passage, a single-cut absorbent main body 10 produced in a separate process (not shown) is fixed in a state of spanning between the two continuous sheets 21a and 22a related to the front waist portion 20 and the two continuous sheets 31a and 32a related to the back waist portion 30, thus forming the substantially ladder-shaped diaper continuous body 1hs in which substantially H-shaped unfolded diapers 1h, 1h, ... are continuous with each other. The fixing of the absorbent main body 10 can be performed, for example, using a rotating drum device (not shown). The rotating drum device has a rotating drum rotating along the direction of transport, for example, which has a plurality of holding portions releasably holding the absorbent main body 10 on its outer circumferential surface.

Next, the approximately ladder-shaped diaper continuous body 1hs passes a third processing position PK3. At the time of the passing, the absorbent main body 10 is folded one time at a predetermined position CL1 of the absorbent main body 10 in the CD direction, and thereby the two continuous sheets 21a and 22a relating to the front waist portion 20 and the two continuous sheets 31a and 32a relating to the back waist portion 30 are overlaid on each other in the thickness direction. The folding can be performed using a fold guiding device (not shown), for example. The fold guiding device has a guide plate and a guide roller that are arranged at predetermined positions in the direction of transport, for example. These guide plates and guide rollers guide the approximately ladder-shaped diaper continuous body 1hs passing at the arrangement position so as to be folded one time.

Next, the diaper continuous body 1hsb in the folded state passes the fourth processing position PK4. At the time of the passing, concerning two continuous sheets 21a and 22a related to the front waist portion 20 and concerning the two continuous sheets 31a and 32a related to the back waist portion 30, the continuous sheets 21a, 22a, 31a and 32a which are overlaid in the thickness direction are welded and joined by an ultrasonic welding device 200 (described later) at positions on both sides of the cutting target position PC in the direction of transport so as to form a pair of the joining portions 70 and 70, thereby fixing the diaper continuous body 1hsb in a folded state. This consequently produces an underpants-shaped diaper continuous body 1s in which a plurality of underpants-shaped diapers 1, 1, ... are connected in the lateral direction.

The joining regions SS have a plurality of the joining portions 70, 70, 70... that are lined up side-by-side in the CD direction (vertical direction). The joining portions 70, 70, 70... weld together the continuous sheet 21a of the front waist portion 20 and the continuous sheet 31a of the back waist portion 30, and also weld together the pair of opposing surfaces 21ast and 22ast of the continuous sheets 21a and 22a of the front waist portion 20, and also weld together the pair of opposing surfaces 31ast and 32ast of the continuous sheets 31a and 32a of the back waist portion 30.

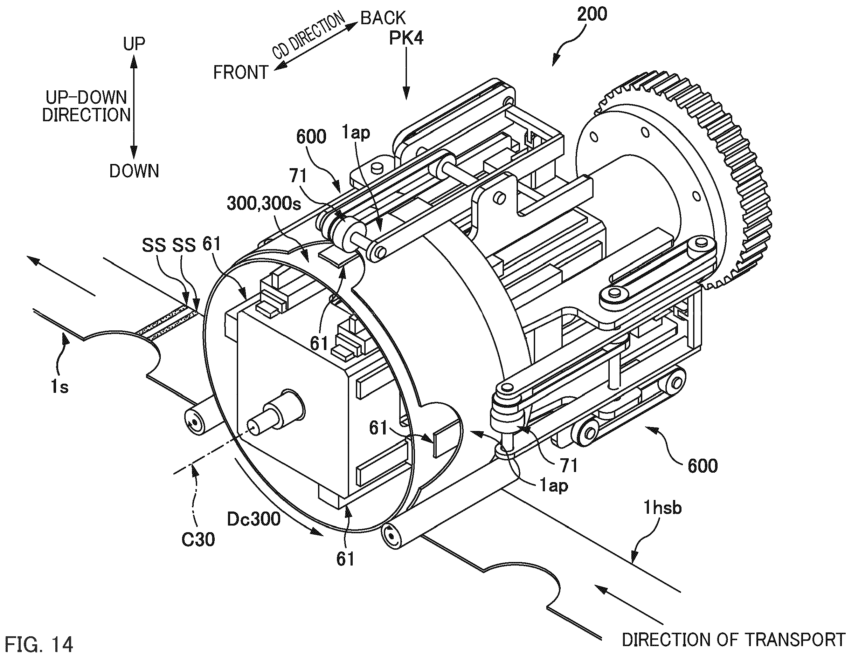

FIG. 14 is a diagram illustrating the processing performed at the fourth processing position PK4. That is, FIG. 14 is a schematic perspective view of the ultrasonic welding device 200, which constitutes the main equipment for that processing, seen from above and diagonally forward.

As shown in FIG. 14, the ultrasonic welding device 200 at the fourth processing position PK4 includes: a rotating drum 300 which has an substantially cylindrical shape and rotates in one direction around a central axis C30 aligned with the CD direction; and a plurality of ultrasonic processing units 600, 600... which rotate together with the rotating drum 300 around the central axis C30. The ultrasonic welding device 200 sends out the diaper continuous body 1hsb transported from the upper process, to the lower process, while winding it with almost no relative slippage between the outer circumferential surface 300s of the rotating drum 300 and the diaper continuous body 1hsb.

Further, the ultrasonic processing units 600, 600,... are provided at predetermined angular intervals (for example, 90°) in the rotation direction Dc300 of the rotating drum 300. Each ultrasonic processing unit 600 includes: a horn 61 which is arranged so as not to be able to move relative to the outer circumferential surface 300s of the rotating drum 300 and which vibrates ultrasonically; and a roller-shaped anvil 71 which is arranged outside the horn 61 in the rotation radial direction Dr300 of the rotating drum 300 so as to clamp the diaper continuous body 1hsb in cooperation with the horn 61.

The horn 61 is provided extending to in the CD direction and has a rail shape. The roller-shaped anvil 71 is capable of rolling along the CD direction relative to this rail-shaped horn 61. This allows the anvil roller 71 to move back and forth in the CD direction relative to the portion 1ap of the diaper continuous body 1hsb that is placed on the horn 61. Therefore, during this reciprocating movement, ultrasonic energy from the horn 61 is selectively applied to the portion 1ap of the diaper continuous body 1hsb that is sandwiched between the anvil roller 71 and the horn 61, forming the joining portions 70 in the above-mentioned portion 1ap of the diaper continuous body 1hsb, and the joining region SS is formed.

In this manner, by using the ultrasonic welding device 200, ultrasonic joining can be performed without any relative movement of both the horn 61 and the anvil 71 with respect to the joining portions 70. Therefore, even when a plurality of small joining portions 70 are to be formed as in the diaper 1, it becomes easier to form the joining portions 70 reliably at accurate positions.

The joining portions 70 may be formed using a heat sealing device (not shown), for example. The heat sealing device has a pair of rolls that are heated while rotating in the direction of transport, for example. One of the rolls is a heat embossing roll that has protrusion portions corresponding to the shapes of the joining portions 70 on the outer circumferential surface, and the other roll is an anvil roll that receives the protrusion portions with a smooth outer circumferential surface.

Next, as shown in FIG. 13, the underpants-shaped-diaper continuous body 1s passes the fifth processing position PK5. At the time of the passing, the continuous body 1s is cut at the cutting target position PC that is located between a pair of the joining portions 70 and 70, thus obtaining the diaper 1.

The cutting can be performed using a cutter device (not shown), for example. A cutter device has a pair of rolls that rotates in the direction of transport, for example. One of the rolls is a cutter roll that has a cutter blade provided on the outer circumferential surface, and the other roll is an anvil roll having an outer circumferential surface that receives the cutter blade.

### Other embodiments

Although the embodiment of the present disclosure has been described hereinabove, the above embodiment of the present disclosure is simply to facilitate understanding of the present disclosure and is not in any way to be construed as limiting the present disclosure.

In the above embodiment, the skin-side sheet 21 and the non-skin-side sheet 22 of the front waist portion 20 are both configured to include the heat-fusible fibers F and the heat-fusing portions EB, but the present inventio is not limited thereto. Either the skin-side sheet 21 (31) or the non-skin-side sheet 22 (32) of the front waist portion 20 (30) may have the heat-fusing portions EB.

Furthermore, in the above embodiment, the front waist portion 20 is torn (cut) after use, but the back waist portion 30 may be torn after use.

### REFERENCE SIGNS LIST

1 diaper (underpants-shaped absorbent article, underpants-shaped disposable diapers),
10 absorbent main body,
11 absorbent core,
12 top sheet, 13 back sheet, 14 exterior sheet,
20 front waist portion (waist portion),
21 skin-side sheet (one-side sheet), 22 non-skin-side sheet (other-side sheet),
23 waist elastic member (elastic member),
30 back waist portion (waist portion), 30E extension portion,
31 skin-side sheet (one-side sheet), 32 non-skin-side sheet (other-side sheet),
33 waist elastic member (elastic member),
50 weld portion, 51 weld-portion pair,
70 joining portion, 70e inner end in lateral direction,
EB heat-fusing portion, R weld-portion row,
SS joining region,
L cutting line,
100 manufacturing line,
200 ultrasonic welding device,
71 anvil (anvil roller), 61 horn,
BH waist opening, LH Leg opening

## Claims

1. An underpants-shaped absorbent article (1) having a vertical direction, a lateral direction, and a front-back direction that intersect each other,
the underpants-shaped absorbent article (1) comprising:
a pair of waist portions (20, 30); and
a liquid-absorbent absorbent body (10),
the pair of waist portions (20, 30) being joined to each other by a pair of joining regions (SS) at both side end portions in the lateral direction,
the joining regions (SS) each having a plurality of joining portions (70) extending along the vertical direction,
the waist portions including a one-side sheet (21) and an other-side sheet (22),
at least either one of the one-side sheet (21) and the other-side sheet (22) having a plurality of heat-fusing portions (EB) in which heat-fusible fibers are joined to each other,
a heat-fusing-portion row (E) extending along the vertical direction being formed by a group of the heat-fusing portions
that are adjacent in the vertical direction in the stretched state and
in which a distance of centers (e) in the lateral direction is smallest in the stretched state,
the heat-fusing portion not joining the one-side sheet (21) and the other-side sheet (22),
the heat-fusing-portion row (E) having a first heat-fusing portion (EB1) and a second heat-fusing portion (EB2) that are located inside of the joining portions (70) in the lateral direction,
a vertical gap (D70) between the joining portions (70) being wider than a vertical length (H70) of the joining portion (70),
the vertical gap (D70) between the joining portions (70) being narrower than a gap (DEB) between the first heat-fusing portion (EB1) and the second heat-fusing portion (EB2).

2. The underpants-shaped absorbent article (1) according to claim 1, wherein
a vertical length (H70) of the joining portion (70) is shorter than a vertical length (HEB) of the first heat-fusing portion (EB1).

3. The underpants-shaped absorbent article (1) according to claim 1 or 2, wherein
a lateral length (W70) of the joining portion (70) is longer than a vertical length (H70) of the joining portion (70).

4. The underpants-shaped absorbent article (1) according to claim 1 or 2, wherein
a plurality of elastic members (23) that can stretch and contract in the lateral direction are arranged at intervals in the vertical direction, between the one-side sheet (21) and the other-side sheet (22), and
a vertical length (H70) of the joining portion (70) is shorter than a thickness of the elastic member (23) in the natural state.

5. The underpants-shaped absorbent article (1) according to claim 1 or 2, wherein
a plurality of elastic members (23) that can stretch and contract in the lateral direction are arranged at intervals in the vertical direction, between the one-side sheet (21) and the other-side sheet (22), and
a vertical length (W70) of the joining portion (70) is shorter than a thickness of the elastic member (23) in the stretched state.

6. The underpants-shaped absorbent article (1) according to claim 1 or 2, wherein
in at least one of the waist portions (20, 30),
a plurality of elastic members (23) that can stretch and contract in the lateral direction are arranged at intervals in the vertical direction, between the one-side sheet (21) and the other-side sheet (22) joined by a plurality of weld portions (70),
the plurality of weld portions (50) include a plurality of weld-portion pairs (51) that are located on both sides of the elastic member (23) in the vertical direction,
when each of the elastic members (23) contracts in the lateral direction,
a position of the elastic member (23) in the vertical direction relative to the one-side sheet (21) and the other-side sheet (22) is restricted by being sandwiched between the weld-portion pair (51), and
a lateral length (W70) of the joining portion (70) is longer than a lateral length (W50) of the weld portion (50).

7. The underpants-shaped absorbent article (1) according to claim 1 or 2, wherein
in at least one of the waist portions (20, 30),
a plurality of elastic members (23) that can stretch and contract in the lateral direction are arranged at intervals in the vertical direction, between the one-side sheet (21) and the other-side sheet (22) joined by a plurality of weld portions (50),
the plurality of weld portions (50) include a plurality of weld-portion pairs (51) that are located on both sides of the elastic member (23) in the vertical direction,
when each of the elastic members (23) contracts in the lateral direction,
a position of the elastic member (23) in the vertical direction relative to the one-side sheet (21) and the other-side sheet (22) is restricted by being sandwiched between the weld-portion pair (51),
the plurality of weld-portion pair (51) includes
a first weld-portion pair (51a1) and
a second weld-portion pair (51a2) located adjacent to the first weld-portion pair (51a1) on a one side in the lateral direction,
the first weld-portion pair (51a1) and the second weld-portion pair (51a2) sandwich a first elastic member (23) between them,
the first weld-portion pair (51a1) has a first weld portion (501) and a second weld portion (502),
the first weld portion (501) and the second weld portion (502) being located on the same side in the vertical direction with respect to the first elastic member (23) and spaced with a first gap (S1) in the lateral direction, and
in the stretched state, a first length (D1) is longer than a lateral length of the first gap (S1),
the first length (D1) being a lateral length from a lateral one-side end of the first weld-portion pair (51a1) to a lateral other-side end of the second weld-portion pair (51a2).

8. The underpants-shaped absorbent article (1) according to claim 1 or 2, wherein
a gap (DEB) between the first heat-fusing portion (EB1) and the second heat-fusing portion (EB2) is greater than twice a sum of a vertical length (H70) of the joining portion (70) and the vertical gap (D70) between the joining portions (70).

9. The underpants-shaped absorbent article (1) according to claim 1 or 2, wherein
the vertical gap (D70) between the joining portions (70) is narrower than a lateral length (W70) of the joining portion (70).

10. The underpants-shaped absorbent article (1) according to claim 1 or 2, wherein
a lateral length (W70) of the joining portion (70) is longer than a lateral length (WEB) of the first heat-fusing portion (EB1) .

11. The underpants-shaped absorbent article (1) according to claim 1 or 2, wherein
an area of the joining portion (70) is equal to or less than an area of the first heat-fusing portion (EB1).

12. The underpants-shaped absorbent article (1) according to claim 1 or 2, wherein
an area of the joining portion (70) is larger than an area of the first heat-fusing portion (EB1).

13. The underpants-shaped absorbent article (1) according to claim 1 or 2, wherein
in at least one of the waist portions (20, 30),
a plurality of elastic members (23) that can stretch and contract in the lateral direction are arranged at intervals in the vertical direction, between the one-side sheet (21) and the other-side sheet (22) joined by a plurality of weld portions (50),
the plurality of weld portions (50) include a plurality of weld-portion pairs (51) that are located on both sides of the elastic member (23) in the vertical direction,
when each of the elastic members (23) contracts in the lateral direction,
a position of the elastic member (23) in the vertical direction relative to the one-side sheet (21) and the other-side sheet (22) is restricted by being sandwiched between the weld-portion pair (51), and
an area of the joining portion (70) is equal to or less than an area of the weld portion.

14. The underpants-shaped absorbent article (1) according to claim 1 or 2, wherein
in at least one of the waist portions (20, 30),
a plurality of elastic members (23) that can stretch and contract in the lateral direction are arranged at intervals in the vertical direction, between the one-side sheet (21) and the other-side sheet (22) joined by a plurality of weld portions (50),
the plurality of weld portions (50) include a plurality of weld-portion pairs (51) that are located on both sides of the elastic member (23) in the vertical direction,
when each of the elastic members (23) contracts in the lateral direction,
a position of the elastic member (23) in the vertical direction relative to the one-side sheet (21) and the other-side sheet (22) is restricted by being sandwiched between the weld-portion pair (51), and
an area of the joining portion (70) is larger than an area of the weld portion.

## Patentansprüche

1. Unterhosen-förmiger absorbierender Artikel (1), der eine vertikale Richtung, eine laterale Richtung und eine Vorn-Hinten-Richtung aufweist, die einander schneiden,
wobei der Unterhosen-förmige absorbierende Artikel (1) Folgendes umfasst:
ein Paar von Taillenteilen (20, 30); und
einen flüssigkeitsabsorbierenden Saugkörper (10),
wobei das Paar von Taillenteilen (20, 30) durch ein Paar von Verbindungsbereichen (SS) an beiden Seitenendteilen in der lateralen Richtung miteinander verbunden sind,
die Verbindungsbereiche (SS) jeweils eine Vielzahl von Verbindungsteilen (70) aufweisen, die sich entlang der vertikalen Richtung erstrecken,
die Taillenteile eine Lage auf einer Seite (21) und eine Lage auf einer anderen Seite (22) einschließen,
mindestens eine der Lage auf der einen Seite (21) und der Lage auf der anderen Seite (22) eine Vielzahl von Wärmeschmelzteilen (EB) aufweist, in denen wärmeschmelzbare Fasern miteinander verbunden sind,
eine Reihe von Wärmeschmelzteilen (E), die sich entlang der vertikalen Richtung erstrecken, durch eine Gruppe der Wärmeschmelzteile gebildet ist,
die in der vertikalen Richtung in dem gedehnten Zustand benachbart sind und
bei denen ein Abstand der Mittelpunkte (e) in der lateralen Richtung in dem gedehnten Zustand am kleinsten ist,
wobei der Wärmeschmelzteil nicht die Lage auf der einen Seite (21) und die Lage auf der anderen Seite (22) verbindet,
die Reihe von Wärmeschmelzteilen (E) einen ersten Wärmeschmelzteil (EB1) und einen zweiten Wärmeschmelzteil (EB2) aufweist, die sich innerhalb der Verbindungsteile (70) in der lateralen Richtung befinden,
eine vertikale Lücke (D70) zwischen den Verbindungsteilen (70) breiter ist als eine vertikale Länge (H70) des Verbindungsteils (70),
die vertikale Lücke (D70) zwischen den Verbindungsteilen (70) schmaler ist als eine Lücke (DEB) zwischen dem ersten Wärmeschmelzteil (EB1) und dem zweiten Wärmeschmelzteil (EB2).

2. Unterhosen-förmiger absorbierender Artikel (1) nach Anspruch 1, wobei
eine vertikale Länge (H70) des Verbindungsteils (70) kürzer ist als eine vertikale Länge (HEB) des ersten Wärmeschmelzteils (EB1).

3. Unterhosen-förmiger absorbierender Artikel (1) nach Anspruch 1 oder 2, wobei
eine laterale Länge (W70) des Verbindungsteils (70) länger ist als eine vertikale Länge (H70) des Verbindungsteils (70).

4. Unterhosen-förmiger absorbierender Artikel (1) nach Anspruch 1 oder 2, wobei
eine Vielzahl von elastischen Elementen (23), die sich in der lateralen Richtung dehnen und zusammenziehen können, in Abständen in der vertikalen Richtung zwischen der Lage auf der einen Seite (21) und der Lage auf der anderen Seite (22) angeordnet ist und
eine vertikale Länge (H70) des Verbindungsteils (70) kürzer ist als eine Dicke des elastischen Elements (23) in dem natürlichen Zustand.

5. Unterhosen-förmiger absorbierender Artikel (1) nach Anspruch 1 oder 2, wobei
eine Vielzahl von elastischen Elementen (23), die sich in der lateralen Richtung dehnen und zusammenziehen können, in Abständen in der vertikalen Richtung zwischen der Lage auf der einen Seite (21) und der Lage auf der anderen Seite (22) angeordnet ist und
eine vertikale Länge (W70) des Verbindungsteils (70) kürzer ist als eine Dicke des elastischen Elements (23) in dem gedehnten Zustand.

6. Unterhosen-förmiger absorbierender Artikel (1) nach Anspruch 1 oder 2, wobei
in mindestens einem der Taillenteile (20, 30)
eine Vielzahl von elastischen Elementen (23), die sich in der lateralen Richtung dehnen und zusammenziehen können, in Abständen in der vertikalen Richtung zwischen der Lage auf der einen Seite (21) und der Lage auf der anderen Seite (22) angeordnet ist, die durch eine Vielzahl von Schweißteilen (70) verbunden sind,
die Vielzahl von Schweißteilen (50) eine Vielzahl von Schweißteilpaaren (51) einschließt, die sich auf beiden Seiten des elastischen Elements (23) in der vertikalen Richtung befinden,
wenn sich jedes der elastischen Elemente (23) in der lateralen Richtung zusammenzieht,
eine Position des elastischen Elements (23) in der vertikalen Richtung in Bezug auf die Lage auf der einen Seite (21) und die Lage auf der anderen Seite (22) durch Einklemmen zwischen dem Schweißteilpaar (51) eingeschränkt ist, und
eine laterale Länge (W70) des Verbindungsteils (70) länger ist als eine laterale Länge (W50) des Schweißteils (50).

7. Unterhosen-förmiger absorbierender Artikel (1) nach Anspruch 1 oder 2, wobei
in mindestens einem der Taillenteile (20, 30)
eine Vielzahl von elastischen Elementen (23), die sich in der lateralen Richtung dehnen und zusammenziehen können, in Abständen in der vertikalen Richtung zwischen der Lage auf der einen Seite (21) und der Lage auf der anderen Seite (22) angeordnet ist, die durch eine Vielzahl von Schweißteilen (50) verbunden sind,
die Vielzahl von Schweißteilen (50) eine Vielzahl von Schweißteilpaaren (51) einschließt, die sich auf beiden Seiten des elastischen Elements (23) in der vertikalen Richtung befinden,
wenn sich jedes der elastischen Elemente (23) in der lateralen Richtung zusammenzieht,
eine Position des elastischen Elements (23) in der vertikalen Richtung in Bezug auf die Lage auf der einen Seite (21) und die Lage auf der anderen Seite (22) durch Einklemmen zwischen dem Schweißteilpaar (51) eingeschränkt ist,
die Vielzahl von Schweißteilpaaren (51) Folgendes einschließt:
ein erstes Schweißteilpaar (51a1) und
ein zweites Schweißteilpaar (51a2), das sich benachbart zu dem ersten Schweißteilpaar (51a1) auf einer Seite in der lateralen Richtung befindet,
das erste Schweißteilpaar (51a1) und das zweite Schweißteilpaar (51a2) ein erstes elastisches Element (23) zwischen ihnen eingeklemmt,
das erste Schweißteilpaar (51a1) einen ersten Schweißteil (501) und einen zweiten Schweißteil (502) aufweist,
wobei sich der erste Schweißteil (501) und der zweite Schweißteil (502) auf der gleichen Seite in der vertikalen Richtung bezüglich des ersten elastischen Elements (23) befindeen und mit einer ersten Lücke (S1) in der lateralen Richtung beabstandet sind, und
in dem gedehnten Zustand eine erste Länge (D1) länger ist als eine laterale Länge der ersten Lücke (S1),
wobei die erste Länge (D1) eine laterale Länge von einem lateralen Ende auf einer Seite des ersten Schweißteilpaars (51a1) zu einem lateralen Ende auf einer anderen Seite des zweiten Schweißteilpaars (51a2) ist.

8. Unterhosen-förmiger absorbierender Artikel (1) nach Anspruch 1 oder 2, wobei
eine Lücke (DEB) zwischen dem ersten Wärmeschmelzteil (EB1) und dem zweiten Wärmeschmelzteil (EB2) größer ist als das Zweifache einer Summe aus einer vertikalen Länge (H70) des Verbindungsteils (70) und der vertikalen Lücke (D70) zwischen den Verbindungsteilen (70).

9. Unterhosen-förmiger absorbierender Artikel (1) nach Anspruch 1 oder 2, wobei
die vertikale Lücke (D70) zwischen den Verbindungsteilen (70) schmaler ist als eine laterale Länge (W70) des Verbindungsteils (70).

10. Unterhosen-förmiger absorbierender Artikel (1) nach Anspruch 1 oder 2, wobei
eine laterale Länge (W70) des Verbindungsteils (70) länger ist als eine laterale Länge (WEB) des ersten Wärmeschmelzteils (EB1).

11. Unterhosen-förmiger absorbierender Artikel (1) nach Anspruch 1 oder 2, wobei
eine Fläche des Verbindungsteils (70) gleich oder kleiner ist als eine Fläche des ersten Wärmeschmelzteils (EB1).

12. Unterhosen-förmiger absorbierender Artikel (1) nach Anspruch 1 oder 2, wobei
eine Fläche des Verbindungsteils (70) größer ist als eine Fläche des ersten Wärmeschmelzteils (EB1).

13. Unterhosen-förmiger absorbierender Artikel (1) nach Anspruch 1 oder 2, wobei
in mindestens einem der Taillenteile (20, 30)
eine Vielzahl von elastischen Elementen (23), die sich in der lateralen Richtung dehnen und zusammenziehen können, in Abständen in der vertikalen Richtung zwischen der Lage auf der einen Seite (21) und der Lage auf der anderen Seite (22) angeordnet ist, die durch eine Vielzahl von Schweißteilen (50) verbunden sind,
die Vielzahl von Schweißteilen (50) eine Vielzahl von Schweißteilpaaren (51) einschließt, die sich auf beiden Seiten des elastischen Elements (23) in der vertikalen Richtung befinden,
wenn sich jedes der elastischen Elemente (23) in der lateralen Richtung zusammenzieht,
eine Position des elastischen Elements (23) in der vertikalen Richtung in Bezug auf die Lage auf der einen Seite (21) und die Lage auf der anderen Seite (22) durch Einklemmen zwischen dem Schweißteilpaar (51) eingeschränkt ist, und
eine Fläche des Verbindungsteils (70) gleich oder kleiner ist als eine Fläche des Schmelzteils.

14. Unterhosen-förmiger absorbierender Artikel (1) nach Anspruch 1 oder 2, wobei
in mindestens einem der Taillenteile (20, 30)
eine Vielzahl von elastischen Elementen (23), die sich in der lateralen Richtung dehnen und zusammenziehen können, in Abständen in der vertikalen Richtung zwischen der Lage auf der einen Seite (21) und der Lage auf der anderen Seite (22) angeordnet ist, die durch eine Vielzahl von Schweißteilen (50) verbunden sind,
die Vielzahl von Schweißteilen (50) eine Vielzahl von Schweißteilpaaren (51) einschließt, die sich auf beiden Seiten des elastischen Elements (23) in der vertikalen Richtung befinden,
wenn sich jedes der elastischen Elemente (23) in der lateralen Richtung zusammenzieht,
eine Position des elastischen Elements (23) in der vertikalen Richtung in Bezug auf die Lage auf der einen Seite (21) und die Lage auf der anderen Seite (22) durch Einklemmen zwischen dem Schweißteilpaar (51) eingeschränkt ist, und
eine Fläche des Verbindungsteils (70) größer ist als eine Fläche des Schmelzteils.

## Revendications

1. Article absorbant en forme de sous-vêtement (1) présentant une direction verticale, une direction latérale et une direction antéropostérieure qui sont en intersection les unes avec les autres,
l'article absorbant en forme de sous-vêtement (1) comprenant :
une paire de portions de ceinture (20, 30) ; et
un corps absorbant qui absorbe les liquides (10),
la paire de portions de ceinture (20, 30) étant jointes l'une à l'autre par une paire de régions de jonction (SS) aux deux positions d'extrémité latérales dans la direction latérale,
les régions de jonction (SS) comportant chacune une pluralité de portions de jonction (70) qui se prolongent le long de la direction verticale,
les portions de ceinture incluant une feuille située sur une face (21) et une feuille située sur l'autre face (22),
une au moins entre la feuille située sur une face (21) et la feuille située sur l'autre face (22) ayant une pluralité de portions de thermofusion (EB) dans lesquelles des fibres thermofusibles sont jointes les unes aux autres,
une rangée constituant une portion de thermofusion (E) qui se prolonge le long de la direction verticale étant formée par un groupe de portions de thermofusion adjacentes dans la direction verticale à l'état étiré, et dans laquelle la distance la plus faible des centres (e) dans la direction latérale est à l'état étiré,
la portion de thermofusion ne joignant ni la feuille située sur une face (21), ni la feuille située sur l'autre face (22),
la rangée constituant une portion de thermofusion (E) comportant une première portion de thermofusion (EB1) et une seconde portion de thermofusion (EB2) localisées à l'intérieur des portions de jonction (70) dans la direction latérale,
un espace vertical (D70) entre les portions de jonction (70) étant plus large qu'une longueur verticale (H70) de la portion de jonction (70),
l'espace vertical (D70) entre les portions de jonction (70) étant plus étroit qu'un espace (DEB) entre la première portion de thermofusion (EB1) et la seconde portion de thermofusion (EB2).

2. Article absorbant en forme de sous-vêtement (1) selon la revendication 1, où
une longueur verticale (H70) de la portion de jonction (70) est plus courte qu'une longueur verticale (HEB) de la première portion de thermofusion (EB1).

3. Article absorbant en forme de sous-vêtement (1) selon la revendication 1 ou 2, où
une longueur latérale (W70) de la portion de jonction (70) est plus élevée qu'une longueur verticale (H70) de la portion de jonction (70).

4. Article absorbant en forme de sous-vêtement (1) selon la revendication 1 ou 2, où
une pluralité d'éléments élastiques (23) qui peuvent s'étirer et se contracter dans la direction latérale sont agencés à intervalles dans la direction verticale entre la feuille située sur une face (21) et la feuille située sur l'autre face (22) et
une longueur verticale (H70) de la portion de jonction (70) est plus courte qu'une épaisseur de l'élément élastique (23) à l'état naturel.

5. Article absorbant en forme de sous-vêtement (1) selon la revendication 1 ou 2, où
une pluralité d'éléments élastiques (23) qui peuvent s'étirer et se contracter dans la direction latérale sont agencés à intervalles dans la direction verticale entre la feuille située sur une face (21) et la feuille située sur l'autre face (22) et
une longueur verticale (W70) de la portion de jonction (70) est plus courte qu'une épaisseur de l'élément élastique (23) à l'état étiré.

6. Article absorbant en forme de sous-vêtement (1) selon la revendication 1 ou 2, où
dans une au moins des portions de ceinture (20, 30),
une pluralité d'éléments élastiques (23) qui peuvent s'étirer et se contracter dans la direction latérale sont agencés à intervalles dans la direction verticale entre la feuille située sur une face (21) et la feuille située sur l'autre face (22) qui sont jointes par une pluralité de portions de soudage (70),
la pluralité de portions de soudage (50) inclut une pluralité de paires de portions de soudage (51) qui sont localisées des deux côtés de l'élément élastique (23) dans la direction verticale,
quand chacun des membres élastiques (23) se contracte dans la direction latérale,
une position de l'élément élastique (23) dans la direction verticale par rapport à la feuille située sur une face (21) et à la feuille située sur l'autre face (22) est limitée du fait de sa localisation en sandwich entre la paire de portions de soudage (51) et
une longueur latérale (W70) de la portion de jonction (70) est plus élevée qu'une longueur latérale (W50) de la portion de soudage (50).

7. Article absorbant en forme de sous-vêtement (1) selon la revendication 1 ou 2, où
dans une au moins des portions de ceinture (20, 30),
une pluralité d'éléments élastiques (23) qui peuvent s'étirer et se contracter dans la direction latérale sont agencés à intervalles dans la direction verticale entre la feuille située sur une face (21) et la feuille située sur l'autre face (22) qui sont jointes par une pluralité de portions de soudage (50),
la pluralité de portions de soudage (50) inclut une pluralité de paires de portions de soudage (51) qui sont localisées des deux côtés de l'élément élastique (23) dans la direction verticale,
quand chacun des membres élastiques (23) se contracte dans la direction latérale,
une position de l'élément élastique (23) dans la direction verticale par rapport à la feuille située sur une face (21) et à la feuille située sur l'autre face (22) est limitée en raison de sa localisation en sandwich entre la paire de portions de soudage (51),
la pluralité de paires de portions de soudage (51) inclut
une première paire de portions de soudage (51a1) et
une seconde paire de portions de soudage (51a2) située en un point adjacent à la première paire de portions de soudage (51a1) sur une face dans la direction latérale,
la première paire de portions de soudage (51a1) et la seconde paire de portions de soudage (51a2) placent en sandwich un premier élément élastique (23) entre elles,
la première paire de portions de soudage (51a1) comporte une première portion de soudage (501) et une seconde portion de soudage (502),
la première portion de soudage (501) et la seconde portion de soudage (502) étant localisées du même côté dans la direction verticale par rapport au premier élément élastique (23) et séparées par un premier espace (S1) dans la direction latérale et
à l'état étiré, une première longueur (D1) est plus élevée qu'une longueur latérale du premier espace (S1),
la première longueur (D1) étant une longueur latérale qui va d'une extrémité latérale sur une face de la première paire de portions de soudage (51a1) à une extrémité latérale sur l'autre face de la seconde paire de portions de soudage (51a2).

8. Article absorbant en forme de sous-vêtement (1) selon la revendication 1 ou 2, où
un espace (DEB) entre la première portion de thermofusion (EB1) et la seconde portion de thermofusion (EB2) est plus large que le double d'une somme d'une longueur verticale (H70) de la portion de jonction (70) et de l'espace vertical (D70) entre les portions de jonction (70).

9. Article absorbant en forme de sous-vêtement (1) selon la revendication 1 ou 2, où
l'espace vertical (D70) entre les portions de jonction (70) est plus étroit qu'une longueur latérale (W70) de la portion de jonction (70).

10. Article absorbant en forme de sous-vêtement (1) selon la revendication 1 ou 2, où
une longueur latérale (W70) de la portion de jonction (70) est plus élevée qu'une longueur latérale (WEB) de la première portion de thermofusion (EB1).

11. Article absorbant en forme de sous-vêtement (1) selon la revendication 1 ou 2, où
une superficie de la portion de jonction (70) est inférieure ou égale à une superficie de la première portion de thermofusion (EB1).

12. Article absorbant en forme de sous-vêtement (1) selon la revendication 1 ou 2, où
une superficie de la portion de jonction (70) est plus élevée qu'une superficie de la première portion de thermofusion (EB1).

13. Article absorbant en forme de sous-vêtement (1) selon la revendication 1 ou 2, où
dans une au moins des portions de ceinture (20, 30),
une pluralité d'éléments élastiques (23) qui peuvent s'étirer et se contracter dans la direction latérale sont agencés à intervalles dans la direction verticale entre la feuille située sur une face (21) et la feuille située sur l'autre face (22) qui sont jointes par une pluralité de portions de soudage (50),
la pluralité de portions de soudage (50) inclut une pluralité de paires de portions de soudage (51) qui sont localisées des deux côtés de l'élément élastique (23) dans la direction verticale,
quand chacun des membres élastiques (23) se contracte dans la direction latérale,
une position de l'élément élastique (23) dans la direction verticale par rapport à la feuille située sur une face (21) et à la feuille située sur l'autre face (22) est limitée en raison de sa localisation en sandwich entre la paire de portions de soudage (51) et
une superficie de la portion de jonction (70) est inférieure ou égale à une superficie de la portion de soudage.

14. Article absorbant en forme de sous-vêtement (1) selon la revendication 1 ou 2, où
dans une au moins des portions de ceinture (20, 30),
une pluralité d'éléments élastiques (23) qui peuvent s'étirer et se contracter dans la direction latérale sont agencés à intervalles dans la direction verticale entre la feuille située sur une face (21) et la feuille située sur l'autre face (22) qui sont jointes par une pluralité de portions de soudage (50),
la pluralité de portions de soudage (50) inclut une pluralité de paires de portions de soudage (51) qui sont localisées des deux côtés de l'élément élastique (23) dans la direction verticale,
quand chacun des membres élastiques (23) se contracte dans la direction latérale,
une position de l'élément élastique (23) dans la direction verticale par rapport à la feuille située sur une face (21) et à la feuille située sur l'autre face (22) est limitée en raison de sa localisation en sandwich entre la paire de portions de soudage (51) et
une superficie de la portion de jonction (70) est supérieure à une superficie de la portion de soudage.
